(19) Europäisches Patentamt
European Patent Office
Office européen des brevets

(11) Veröffentlichungsnummer: **0 016 323**
**B1**

# (12) EUROPÄISCHE PATENTSCHRIFT

(45) Veröffentlichungstag der Patentschrift: **29.09.82**

(51) Int. Cl.³: **C 07 D 249/08**, A 01 N 43/64

(21) Anmeldenummer: **80100535.6**

(22) Anmeldetag: **04.02.80**

(54) **1-Allyltriazol-Derivate, Verfahren zu ihrer Herstellung und ihre Verwendung als Pflanzenschutzmittel.**

(30) Priorität: **16.02.79 DE 2905981**

(43) Veröffentlichungstag der Anmeldung: **01.10.80 Patentblatt 80/20**

(45) Bekanntmachung des Hinweises auf die Patenterteilung: **29.09.82 Patentblatt 82/39**

(84) Benannte Vertragsstaaten: **AT BE CH DE FR GB IT NL SE**

(56) Entgegenhaltungen: **DE-A-2 652 313**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Reiser, Wolf, Dr., Kiebitzweg 17, D-5600 Wuppertal-1 (DE)**
Erfinder: **Draber, Wilfried, Dr., In den Birken 81, D-5600 Wuppertal-1 (DE)**
Erfinder: **Büchel, Karl Heinz, Dr., Bergerheide 62, D-5600 Wuppertal-1 (DE)**
Erfinder: **Lürssen, Klaus, Dr., August-Kierspel-Strasse 89, D-5070 Bergisch-Gladbach (DE)**
Erfinder: **Frohberger, Paul-Ernst, Dr., Willi-Baumeister-Strasse 5, D-5090 Leverkusen (DE)**
Erfinder: **Paul, Volker, Dr., Ahornstrasse 5, D-5650 Solingen (DE)**

EP 0 016 323 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1-Allyltriazol-Derivate, Verfahren zu ihrer Herstellung und ihre Verwendung als Pflanzenschutzmittel

Die vorliegende Erfindung betrifft neue 1-Allyltriazol-Derivate, mehrere Verfahren zu ihrer Herstellung sowie ihre Verwendung als Pflanzenschutzmittel.

Es ist bereits bekannt geworden, dass acylierte und carbamoylierte Derivate von im Phenylteil substituierten 3,3-Dimethyl-1-phenoxy-1-triazolyl-butan-2-olen eine gute fungizide Wirksamkeit besitzen (vergleiche DE-OS 2 600 799 [LeA 16 838]); ebenso bestimmte im Phenylteil substituierte 4,4-Dimethyl-1-phenyl-2-triazolyl-pentan-3-one, wie z.B. 1-(4-Chlorphenyl)-4,4-dimethyl-2-(1,2,4-triazol-1-yl)-pentan-3-on (vergleiche DEOS 2 734 426). Die Wirkung dieser Azol-Derivate ist jedoch, insbesondere bei niedrigen Aufwandmengen und -konzentrationen, nicht immer ganz befriedigend.

Es ist ferner bekannt geworden, dass bestimmte 2-Halogen-ethyl-triazolylammonium-halogenide pflanzenwachstumsregulierende Eigenschaften aufweisen (vergleiche US-Patentschrift 3 156 554). So lässt sich z.B. mit Hilfe von 2-Chlorethyl-trimethyl-ammoniumchlorid eine Beeinflussung des Pflanzenwachstums, insbesondere eine Hemmung des vegetativen Pflanzenwachstums bei wichtigen Kulturpflanzen erzielen. Allerdings ist die Wirksamkeit dieses Stoffes, vor allem bei niedrigen Aufwandmengen, nicht immer ausreichend.

Es ist weiterhin bekannt geworden, dass die 2-Chlorethylphosphonsäure eine wachstumsregulierende Wirkung aufweist (vergleiche DE-AS 1 667 968). Die mit dieser Substanz erzielten Ergebnisse sind jedoch ebenfalls nicht immer zufriedenstellend.

Es wurden nun die neuen 1-Allyltriazol-Derivate der allgemeinen Formel

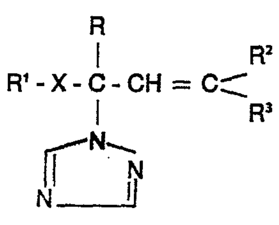

(I)

in welcher

R für Wasserstoff, Alkyl mit 1 bis 4 Kohlenstoffatomen oder für gegebenenfalls durch Halogen substituiertes Benzyl steht;

$R^1$ für Alkyl mit 1 bis 4 Kohlenstoffatomen steht, welches gegebenenfalls substituiert sein kann durch Halogen, Alkylcarbonyloxy- und Alkylsulfonyloxy-Gruppen mit jeweils 1 bis 4 Kohlenstoffatomen im Alkylteil, sowie durch die Phenylsulfonyloxy-Gruppe, wobei letztere selbst durch Alkyl mit 1 bis 4 Kohlenstoffatomen substituiert sein kann; ausserdem für Cycloalkyl mit 5 bis 7 Kohlenstoffatomen und für Phenyl steht, welches substituiert sein kann durch Halogen, Alkyl mit 1 bis 4 Kohlenstoffatomen, Phenyl, Phenoxy, Halogenphenyl und Halogenphenoxy;

$R^2$ für Alkyl mit 1 bis 4 Kohlenstoffatomen steht;

$R^3$ für Alkyl mit 1 bis 4, Cycloalkyl und Cycloalkenyl mit jeweils 5 bis 7 Kohlenstoffatomen steht, wobei letzteres durch Alkyl mit 1 bis 4 Kohlenstoffatomen substituiert sein kann, ferner für Alkenyl mit 2 bis 4 Kohlenstoffatomen und für Phenyl steht, wobei letzteres durch Halogen und Alkyl mit 1 bis 4 Kohlenstoffatomen substituiert sein kann;

$R^2$ und $R^3$ können weiterhin gemeinsam mit dem Kohlenstoffatom, an das sie gebunden sind, für gegebenenfalls durch Alkyl mit 1 bis 4 Kohlenstoffatomen substituiertes Cycloalkenyl mit 5 bis 7 Kohlenstoffatomen und für Cycloalkyl mit 3 bis 7 Kohlenstoffatomen stehen;

X für die Gruppe $-C(OR^4)R^5-$ und für die Ketogruppe steht, wobei

$R^4$ für Wasserstoff, für Alkyl mit 1 bis 4 Kohlenstoffatomen sowie für Benzyl und für Naphthylmethyl steht, wobei die beiden letztgenannten Gruppen durch Halogen, durch Alkyl mit 1 bis 4 Kohlenstoffatomen, durch Phenyl, Phenoxy, Halogenphenyl und Halogenphenoxy substituiert sein können; weiterhin für den Acylrest $-CO-R^{10}$ und den Carbamoylrest $-CO-NR^{11}R^{12}$ steht;

$R^5$ für Wasserstoff, für Alkyl mit 1 bis 4 Kohlenstoffatomen, sowie für gegebenenfalls durch Halogen substituiertes Benzyl steht;

$R^{10}$ für Alkyl mit 1 bis 4 Kohlenstoffatomen, Halogenalkyl mit 1 bis 2 Kohlenstoff- und 1 bis 5 Halogenatomen und für Phenyl und Benzyl steht, wobei die beiden letztgenannten Gruppen durch Halogen und Alkyl mit 1 bis 4 Kohlenstoffatomen substituiert sein können;

$R^{11}$ für Wasserstoff und Alkyl mit 1 bis 4 Kohlenstoffatomen steht; und

$R^{12}$ für Alkyl mit 1 bis 8 Kohlenstoffatomen, Halogenalkyl mit bis zu 4 Kohlenstoff- und bis zu 5 Halogenatomen und für gegebenenfalls halogen-substituiertes Phenyl steht, und schliesslich für Halogenalkylmercapto-Gruppen mit bis zu 2 Kohlenstoff- und bis zu 5 Halogenatomen steht,

sowie deren pflanzenverträglichen Säureadditions-Salze und Metallsalz-Komplexe gefunden.

Die Verbindungen der Formel (I) können gegebenenfalls in zwei geometrischen Isomerenformen vorliegen, je nach Anordnung der Gruppen, die an die Doppelbindung gebunden sind. Wenn X für die Gruppe $-C(OR^4)R^5-$ steht, liegen zwei asymmetrische Kohlenstoffatome vor, so dass die Verbindungen der Formel (I) in zwei diastereomeren Formen und vier optischen Isomeren vorliegen. Sowohl die einzelnen Isomeren als auch die Gemische beider werden erfindungsgemäss beansprucht.

Weiterhin wurde gefunden, dass man 1-Allyltriazol-Derivate der Formel (I) sowie deren pflanzenverträgliche Säureadditions-Salze und Metallsalz-Komplexe erhält, wenn man

a) Triazol-Ketone der Formel

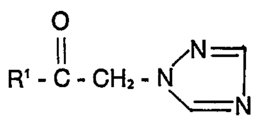

(II)

in welcher

$R^1$ die oben angegebene Bedeutung hat, mit Aldehyden der Formel

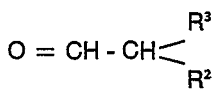

(III)

in welcher

$R^2$ und $R^3$ die oben angegebene Bedeutung haben,

in Gegenwart eines Lösungsmittels und in Gegenwart eines Katalysators umsetzt und das gewünschte Produkt der Formel

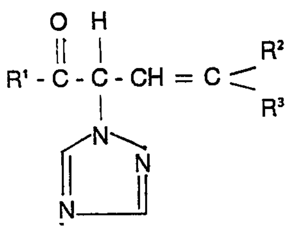

(Ia)

in welcher

$R^1$, $R^2$ und $R^3$ die oben angegebene Bedeutung, haben, isoliert, oder

b) die nach Verfahren (a) erhältlichen Verbindungen der Formel (Ia) in an sich bekannter Art und Weise

α) mit komplexen Hydriden in Gegenwart eines Lösungsmittels reduziert, oder

β) mit einer Grignard-Verbindung der Formel

$$R^6 - Mg - Hal \qquad (IV)$$

in welcher

$R^6$ die oben unter $R^5$ genannten Bedeutungen mit Ausnahme von Wasserstoff besitzt und

Hal für Halogen steht,

in Gegenwart eines Lösungsmittels umsetzt, oder die nach Verfahren (b) erhältlichen Verbindungen der Formel

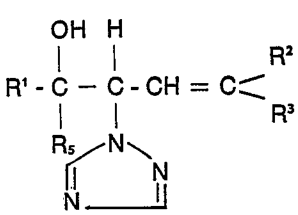

(Ib)

in welcher

$R^1$, $R^2$, $R^3$ und $R^5$ die oben angegebene Bedeutung haben,

c) mit Halogeniden der Formel

$$R^7 - Hal \qquad (V)$$

in welcher

$R^7$ die oben unter $R^4$ genannten Bedeutungen mit Ausnahme von Wasserstoff besitzt, und

Hal für Halogen steht,

in Gegenwart eines Lösungsmittels und gegebenenfalls in Gegenwart einer starken Base bzw. gegebenenfalls in Gegenwart eines Säurebindemittels umsetzt, oder

d) mit Säureanhydriden der Formel

$$R^8 - O - R^8 \qquad (VI)$$

in welcher

$R^8$ für den Azylrest -CO-$R^{10}$ steht, wobei

$R^{10}$ die oben angegeben Bedeutungen besitzt,

in Gegenwart eines Lösungsmittels und gegebenenfalls in Gegenwart eines Katalysators umsetzt, oder

e) mit Isocyanaten der Formel

$$O = C = N - R^9 \qquad (VII)$$

in welcher

$R^9$ für die oben unter $R^{12}$ genannten Bedeutungen mit Ausnahme von Halogenalkylmercapto steht,

in Gegenwart eines Lösungsmittels und gegebenenfalls in Gegenwart eines Katalysators umsetzt, oder

f) die nach Verfahren (a) erhältlichen Verbindungen der Formel (Ia) mit Halogeniden der Formel

$$Y - Hal \qquad (VIII)$$

in welcher

Y die oben unter R genannten Bedeutungen mit Ausnahme von Wasserstoff besitzt, und

Hal für Halogen steht,

in Gegenwart einer starken Base und in Gegenwart eines Lösungsmittels umsetzt und

gegebenenfalls die erhaltenen Verbindungen der Formel

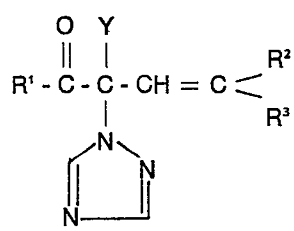

(Iaa)

in welcher

$R^1$, $R^2$, $R^3$ und Y die oben angegebene Bedeutung haben,

entsprechend den Verfahren (b), (c), (d) und (e) weiter umsetzt,

und gegebenenfalls anschliessend eine Säure oder ein Metallsalz addiert.

Die neuen 1-Allyltriazol-Derivate der Formel (I) sowie deren pflanzenverträgliche Säureadditions-Salze und Metallsalz-Komplexe weisen starke fungizide und pflanzenwachstumsregulierende Eigenschaften auf und sind daher als Pflanzenschutzmittel verwendbar.

Überraschenderweise zeigen die erfindungsgemäss verwendbaren 1-Allyltriazol-Derivate eine

bessere fungizide Wirkung, als die aus dem Stand der Technik bekannten acylierten und carbamoylierten Derivate von im Phenylteil substituierten 3,3-Dimethyl-1-triazolyl-butan-2-olen und als das ebenfalls bekannte 1-(4-Chlorphenyl)-4,4-dimethyl-2-(1,2,4-triazol-1-yl)-pentan-3-on, die chemisch und wirkungsmässig naheliegende Verbindungen sind.

Ferner zeigen die erfindungsgemässen verwendbaren Allyltriazol-Derivate sowie deren pflanzenverträglichen Säureadditions-Salze und Metallsalz-Komplexe eine bessere pflanzenwachstumsregulierende Wirkung als das bekannte 2-Chlorethyl-trimethylammoniumchlorid und als die ebenfalls bekannte 2-Chlorethylphosphonsäure, welches anerkannt gut wirksame Stoffe gleicher Wirkungsart sind. Die erfindungsgemässen Stoffe stellen somit eine wertvolle Bereicherung der Technik dar.

Die erfindungsgemässen 1-Allyltriazol-Derivate sind durch die Formel (I) allgemein definiert.

Bevorzugt sind diejenigen Verbindungen der Formel (I), in denen R für Wasserstoff, Methyl, Ethyl, Benzyl, Chlorbenzyl oder Dichlorbenzyl steht; $R^1$ für tert.-Butyl, Isopropyl, Chlor-tert.-butyl, Brom-tert.-butyl; Fluor-tert.-butyl, Acetoxy-tert.-butyl, Methylsulfonyl-oxy-tert.-butyl, p-Toluolsulfonyloxy-tert-butyl; 1,3-Dichlor-2-methyl-prop-2-yl; 1,3-Dibrom-2-methyl-prop-2-yl; 1,3-Difluor-2-methyl-prop-2-yl; 1-Chlor-3-brom-2-methyl-prop-2-yl; 1,3-Diacetoxy-2-methyl-prop-2-yl; Cyclohexyl, Phenyl, Chlorphenyl, Bromphenyl, Dichlorphenyl, Fluorphenyl, Methylphenyl, Dimethylphenyl, Chlor-methylphenyl, Biphenylyl, Phenoxyphenyl, Chlorphenylphenyl oder Chlorphenoxyphenyl steht; $R^2$ für Methyl, Ethyl, Propyl oder Butyl steht; $R^3$ für Methyl, Ethyl, Isopropyl, Cyclohexyl, Cyclohexenyl, Methylcyclohexenyl, Allyl, Methacryl, Phenyl, Chlorphenyl, Dichlorphenyl oder Methylphenyl steht; $R^2$ und $R^3$ gemeinsam mit dem Kohlenstoffatom, an das sie gebunden sind, für Cyclopropyl, Cyclobutyl, Cyclopentyl, Cyclohexyl, Cyclohexenyl oder Methylcyclohexenyl stehen; X für die Gruppe $-C(OR^4)R^5-$ sowie für die Ketogruppe steht; $R^4$ für Wasserstoff, Methyl, Ethyl, n-Propyl, Isopropyl, Isobutyl, gegebenenfalls durch Chlor substituiertes Naphthylmethyl, gegebenenfalls einfach oder mehrfach, gleich oder verschieden durch Chlor, Fluor, Methyl, Phenyl, Chlorphenyl, Phenoxy, Chlorphenoxy substituiertes Benzyl, den Acylrest $-CO-R^{10}$ oder den Carbamoylrest $-CO-NR^{11}R^{12}$ steht; $R^5$ für Wasserstoff, Methyl, Ethyl, Isopropyl, Benzyl, Chlorbenzyl oder Dichlorbenzyl steht; $R^{10}$ für Methyl, Ethyl, Isopropyl, Isobutyl, Chlormethyl, Dichlormethyl oder gegebenenfalls einfach oder mehrfach substituiertes Phenyl und Benzyl mit Chlor, Brom oder Methyl als Substituenten steht; $R^{11}$ für Wasserstoff, Methyl oder Ethyl steht und $R^{12}$ für Methyl, Ethyl, Chlorethyl, Phenyl, Chlorphenyl, Trifluormethyl-, Chlordifluormethyl-, Dichlorfluormethyl- oder Trichlormethyl-mercapto steht.

Im einzelnen seien ausser den bei den Herstellungsbeispielen genannten Verbindungen folgende Verbindungen der allgemeinen Formel (I) genannt:

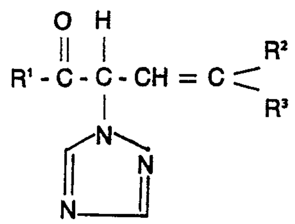

(Ia)

| $R^1$ | $R^2$ | $R^3$ |
|---|---|---|
| $C(CH_3)_3$ | $C_2H_5$ | $C_2H_5$ |
| $C(CH_3)_3$ | $C_2H_5$ | $CH_3$ |
| $C(CH_3)_3$ | $CH_3$ | $CH_3$ |
| $C(CH_3)_3$ | $CH_3$ | 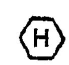 |
| $C(CH_3)_3$ | $CH_3$ | 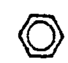 |
| $C(CH_3)_3$ | Cyclopropyl | |
| $C(CH_3)_3$ | Cyclobutyl | |
| $C(CH_3)_3$ | Cyclopentyl | |
| $C(CH_3)_3$ | Cycloheptyl | |
| $C(CH_3)_3$ | Norbon-3-en-2-yl | |

| $R^1$ | $R^2$ | $R^3$ |
|---|---|---|
| 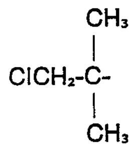 | Cyclohexan | |
| 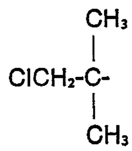 | Cyclohexen | |
| 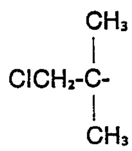 | Methylcyclohexen | |
| 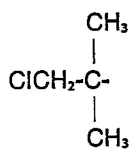 | $CH_3$ | $CH_3$ |
| 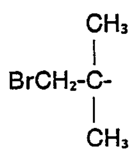 | Cyclohexan | |
| 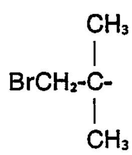 | Cyclohexen | |
| 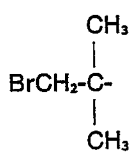 | Methylcyclohexen | |
| 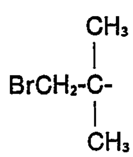 | Methylcyclohexen | |
| 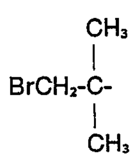 | $CH_3$ | $CH_3$ |
| 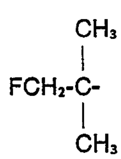 | Cyclohexan | |

| $R^1$ | $R^2$ | $R^3$ |
|---|---|---|
| 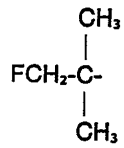 | Cyclohexen | |
| 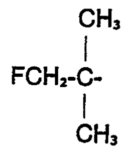 | Methylcyclohexen | |
| 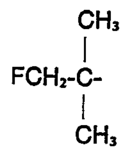 | $CH_3$ | $CH_3$ |
| 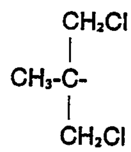 | Cyclohexan | |
| 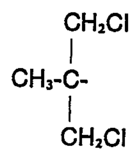 | Cyclohexen | |
| 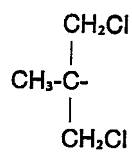 | Methylcyclohexen | |
| 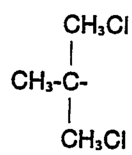 | $CH_3$ | $CH_3$ |
| 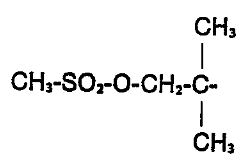 | Cyclohexan | |
| 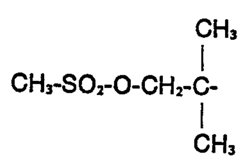 | Cyclohexen | |
| 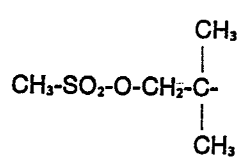 | Methylcyclohexen | |

| $R^1$ | $R^2$ | $R^3$ |
|---|---|---|
| 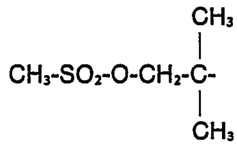 | $CH_3$ | $CH_3$ |
| 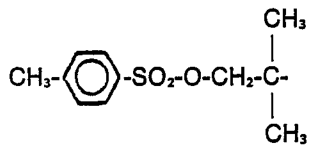 | Cyclohexan | |
| 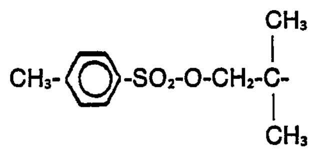 | Cyclohexen | |
| 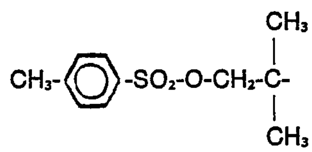 | Methylcyclohexen | |
| 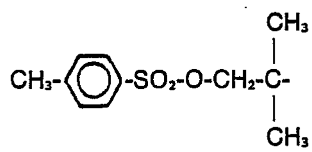 | $CH_3$ | $CH_3$ |
| 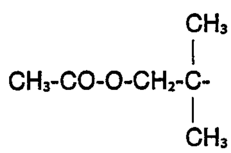 | Cyclohexan | |
| 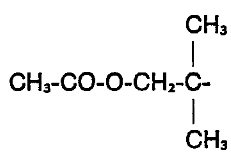 | Cyclohexen | |
| 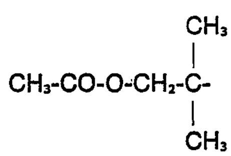 | Methylcyclohexen | |
| 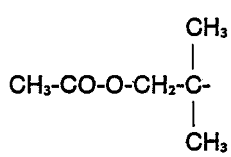 | $CH_3$ | $CH_3$ |
| 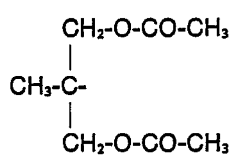 | Cyclohexan | |

| $R^1$ | $R^2$ | $R^3$ |
|---|---|---|
| 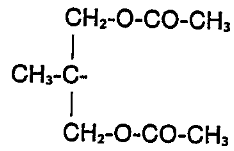 | Cyclohexen | |
| 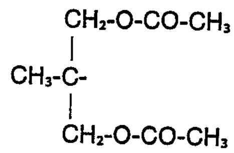 | Methylcyclohexen | |
| 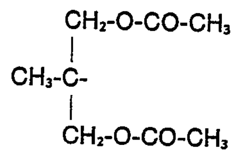 | $CH_3$ | $CH_3$ |
| 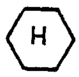 | Cyclohexan | |
| 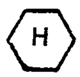 | Cyclohexen | |
| 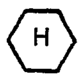 | Methylcyclohexen | |
| 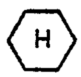 | $CH_3$ | $CH_3$ |

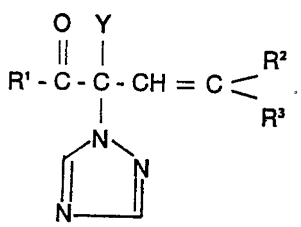

(Iaa)

| $R^1$ | $R^2$ | $R^3$ | Y |
|---|---|---|---|
| $C(CH_3)_3$ | $C_2H_5$ | $CH_3$ | $CH_3$ |
| $C(CH_3)_3$ | $C_2H_5$ | $C_2H_5$ | $CH_3$ |
| $C(CH_3)_3$ | Cyclohexan | | $CH_3$ |
| $C(CH_3)_3$ | Cyclohexen | | $CH_3$ |
| $C(CH_3)_3$ | $C_2H_5$ | $CH_3$ | $C_2H_5$ |
| $C(CH_3)_3$ | $C_2H_5$ | $C_2H_5$ | $C_2H_5$ |
| $C(CH_3)_3$ | Cyclohexan | | $C_2H_5$ |
| $C(CH_3)_3$ | Cyclohexen | | $C_2H_5$ |
| $C(CH_3)_3$ | $C_2H_5$ | $CH_3$ | $-CH_2-$ 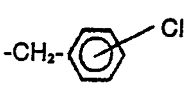 |

| $R^1$ | $R^2$ | $R^3$ | Y |
|---|---|---|---|
| $C(CH_3)_3$ | $C_2H_5$ | $C_2H_5$ | 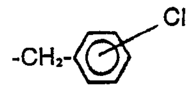 |
| $C(CH_3)_3$ | Cyclohexan | | 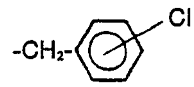 |
| $C(CH_3)_3$ | Cyclohexen | | 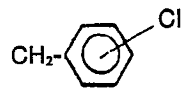 |
| 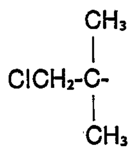 | $CH_3$ | $CH_3$ | $C_2H_5$ |
| 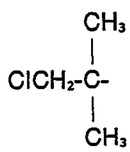 | Cyclohexan | | $C_2H_5$ |
| 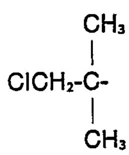 | Cyclohexen | | $C_2H_5$ |
| 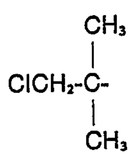 | $CH_3$ | $CH_3$ | 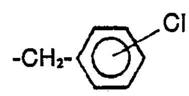 |
| 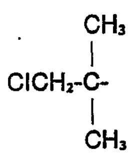 | Cyclohexan | | 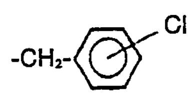 |
| 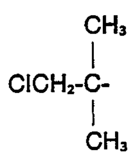 | Cyclohexen | | 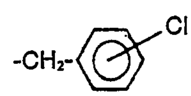 |
| 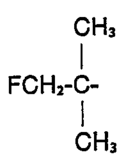 | $CH_3$ | $CH_3$ | $C_2H_5$ |
| 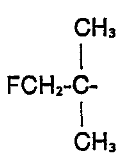 | Cyclohexan | | $C_2H_5$ |

| R¹ | R² | R³ | Y |
|---|---|---|---|
| 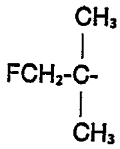 | Cyclohexen | | $C_2H_5$ |
| 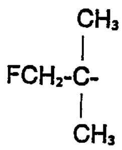 | $CH_3$ | $CH_3$ | 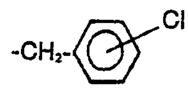 |
| 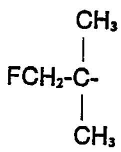 | Cyclohexan | | 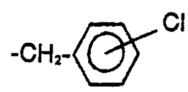 |
| 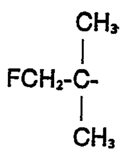 | Cyclohexen | | 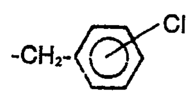 |
| 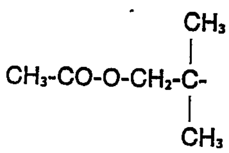 | $CH_3$ | $CH_3$ | $C_2H_5$ |
| 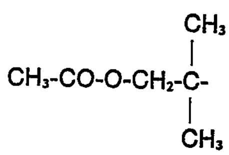 | Cyclohexan | | $C_2H_5$ |
| 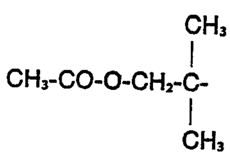 | Cyclohexen | | $C_2H_5$ |
| 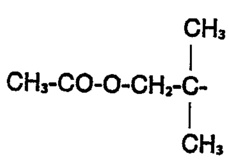 | $CH_3$ | $CH_3$ | 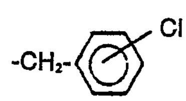 |
| 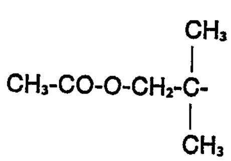 | Cyclohexan | | 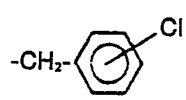 |
| 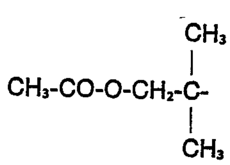 | Cyclohexen | | 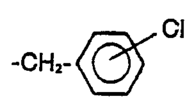 |

| $R^1$ | $R^2$ | $R^3$ | Y |
|---|---|---|---|
| 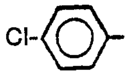 | $CH_3$ | $CH_3$ | $C_2H_5$ |
| 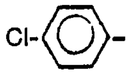 | Cyclohexan | | $C_2H_5$ |
| 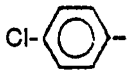 | Cyclohexen | | $C_2H_5$ |
| 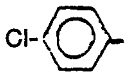 | $CH_3$ | $CH_3$ | 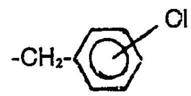 |
| 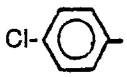 | Cyclohexan | | 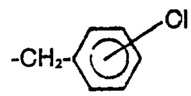 |
| 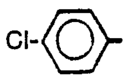 | Cyclohexen | | 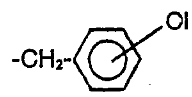 |
| 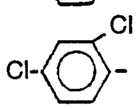 | $CH_3$ | $CH_3$ | $C_2H_5$ |
| 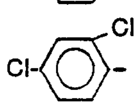 | Cyclohexan | | $C_2H_5$ |
| 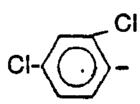 | Cyclohexen | | $C_2H_5$ |
| 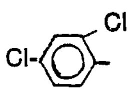 | $CH_3$ | $CH_3$ | 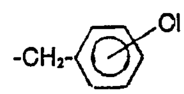 |
| 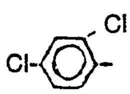 | Cyclohexan | | 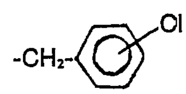 |
| 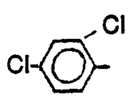 | Cyclohexen | | 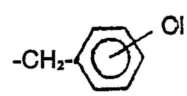 |

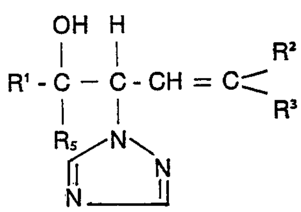

(Ib)

| $R^1$ | $R^2$ | $R^3$ | $R^5$ |
|---|---|---|---|
| $C(CH_3)_3$ | $C_2H_5$ | $CH_3$ | H |
| $C(CH_3)_3$ | $CH_3$ | $CH_3$ | H |

| R¹ | R² | R³ | R⁵ |
|---|---|---|---|
| $C(CH_3)_3$ | $CH_3$ | 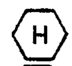 | H |
| $C(CH_3)_3$ | $CH_3$ | 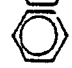 | H |
| $C(CH_3)_3$ | Cyclopropyl | | H |
| $C(CH_3)_3$ | Cyclobutyl | | H |
| $C(CH_3)_3$ | Cyclopentyl | | H |
| $C(CH_3)_3$ | Cycloheptyl | | H |
| $C(CH_3)_3$ | $CH_3$ | $CH_3$ | $CH_3$ |
| $C(CH_3)_3$ | Cyclohexan | | $CH_3$ |
| $C(CH_3)_3$ | Cyclohexen | | $CH_3$ |
| $C(CH_3)_3$ | Methylcyclohexen | | $CH_3$ |
| $C(CH_3)_3$ | $CH_3$ | $CH_3$ | $-CH_2-$ 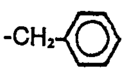 |
| $C(CH_3)_3$ | Cyclohexan | | $-CH_2-$ 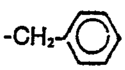 |
| $C(CH_3)_3$ | Cyclohexen | | $-CH_2-$ 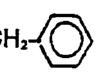 |
| $C(CH_3)_3$ | Methylcyclohexen | | $-CH_2-$ 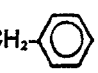 |
| 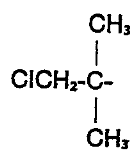 | $CH_3$ | $CH_3$ | H |
| 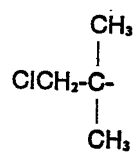 | Cyclohexan | | H |
| 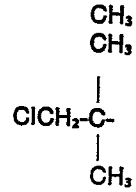 | Cyclohexen | | H |
| 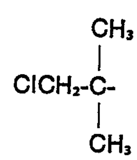 | Methylcyclohexen | | H |
| 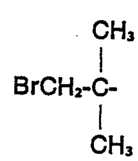 | $CH_3$ | $CH_3$ | H |

| $R^1$ | $R^2$ | $R^3$ | $R^5$ |
|---|---|---|---|
| 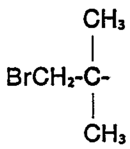 | Cyclohexan | | H |
| 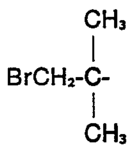 | Cyclohexen | | H |
| 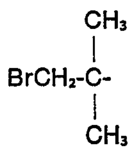 | Methylcyclohexen | | H |
| 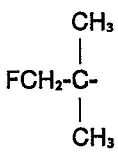 | $CH_3$ | $CH_3$ | H |
| 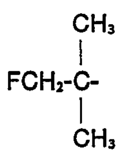 | Cyclohexan | | H |
| 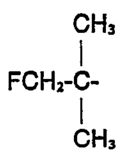 | Cyclohexen | | H |
| 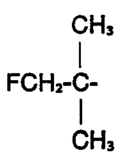 | Methylcyclohexen | | H |
| 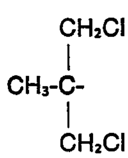 | $CH_3$ | $CH_3$ | H |
| 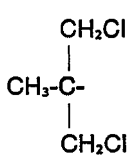 | Cyclohexan | | H |
| 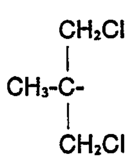 | Cyclohexen | | H |

| $R^1$ | $R^2$ | $R^3$ | $R^5$ |
|---|---|---|---|
| 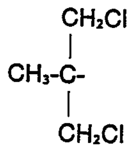 | Methylcyclohexen | | H |
| 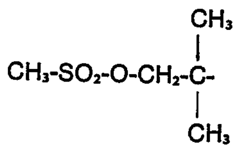 | $CH_3$ | $CH_3$ | H |
| 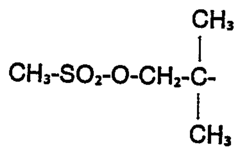 | Cyclohexan | | H |
| 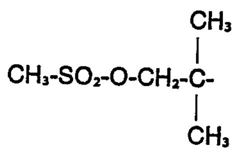 | Cyclohexen | | H |
| 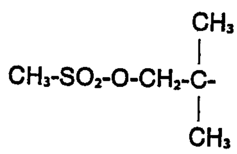 | Methylcyclohexen | | H |
| 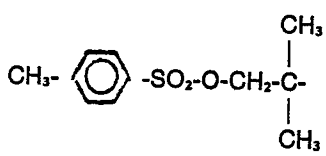 | $CH_3$ | $CH_3$ | H |
| 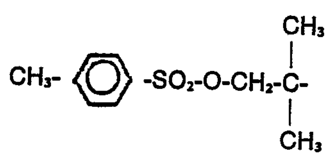 | Cyclohexan | | H |
| 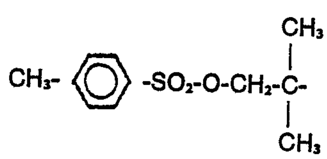 | Cyclohexen | | H |
| 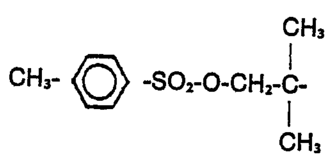 | Methylcyclohexen | | H |
| 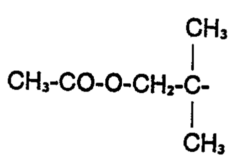 | $CH_3$ | $CH_3$ | H |

| $R^1$ | $R^2$ | $R^3$ | $R^5$ |
|---|---|---|---|
| 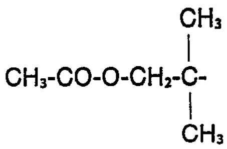 | Cyclohexan | | H |
| 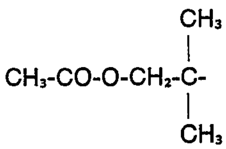 | Cyclohexen | | H |
| 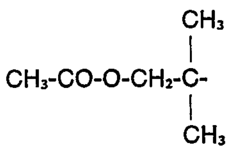 | Methylcyclohexen | | H |
| 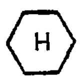 | $CH_3$ | $CH_3$ | H |
| 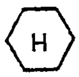 | Cyclohexan | | H |
| 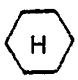 | Cyclohexen | | H |
| 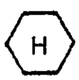 | Methylcyclohexen | | H |
| 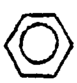 | $CH_3$ | $CH_3$ | H |
| 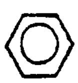 | Cyclohexan | | H |
| 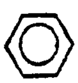 | Cyclohexen | | H |
| 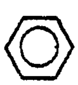 | Methylcyclohexen | | H |
| 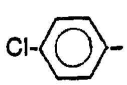 | $CH_3$ | $CH_3$ | H |
| 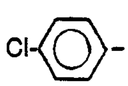 | Cyclohexan | | H |
| 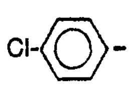 | Cyclohexen | | H |
| 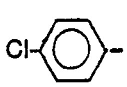 | Methylcyclohexen | | H |

| $R^1$ | $R^2$ | $R^3$ | $R^5$ |
|---|---|---|---|
| 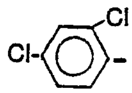 | $CH_3$ | $CH_3$ | H |
| 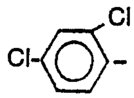 | Cyclohexan | | H |
| 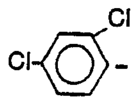 | Cyclohexen | | H |
| 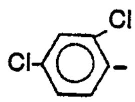 | Methylcyclohexen | | H |

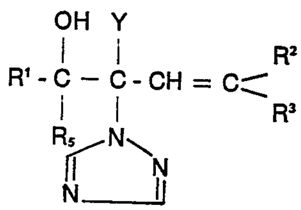

(Ibb)

| $R^1$ | $R^2$ | $R^3$ | $R^5$ | Y |
|---|---|---|---|---|
| $C(CH_3)_3$ | $CH_3$ | $CH_3$ | H | $C_2H_5$ |
| $C(CH_3)_3$ | $C_2H_5$ | $CH_3$ | H | $C_2H_5$ |
| $C(CH_3)_3$ | $C_2H_5$ | $C_2H_5$ | H | $C_2H_5$ |
| $C(CH_3)_3$ | Cyclohexan | | H | $C_2H_5$ |
| $C(CH_3)_3$ | Cyclohexen | | H | $C_2H_5$ |
| $C(CH_3)_3$ | $CH_3$ | $CH_3$ | $CH_3$ | $C_2H_5$ |
| $C(CH_3)_3$ | $C_2H_5$ | $CH_3$ | $CH_3$ | $C_2H_5$ |
| $C(CH_3)_3$ | $C_2H_5$ | $C_2H_5$ | $CH_3$ | $C_2H_5$ |
| $C(CH_3)_3$ | Cyclohexan | | $CH_3$ | $C_2H_5$ |
| $C(CH_3)_3$ | Cyclohexen | | $CH_3$ | $C_2H_5$ |
| $C(CH_3)_3$ | $CH_3$ | $CH_3$ | H | $-CH_2-$ 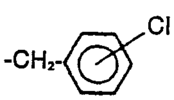 |
| $C(CH_3)_3$ | $C_2H_5$ | $CH_3$ | H | $-CH_2-$ 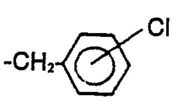 |
| $C(CH_3)_3$ | $C_2H_5$ | $C_2H_5$ | H | $-CH_2-$ 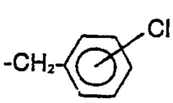 |

| $R^1$ | $R^2$ | $R^3$ | $R^5$ | Y |
|---|---|---|---|---|
| $C(CH_3)_3$ | Cyclohexan | | H | $-CH_2-$ 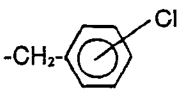 |
| $C(CH_3)_3$ | Cyclohexen | | H | $-CH_2-$ 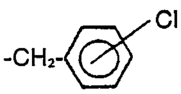 |
| $C(CH_3)_3$ | $CH_3$ | $CH_3$ | $CH_3$ | $-CH_2-$ 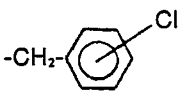 |
| $C(CH_3)_3$ | $C_2H_5$ | $CH_3$ | $CH_3$ | $-CH_2-$ 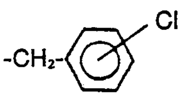 |
| $C(CH_3)_3$ | $C_2H_5$ | $C_2H_5$ | $CH_3$ | $-CH_2-$ 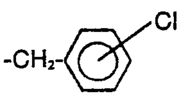 |
| $C(CH_3)_3$ | Cyclohexan | | $CH_3$ | $-CH_2-$ 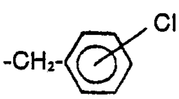 |
| $C(CH_3)_3$ | Cyclohexen | | $CH_3$ | $-CH_2-$ 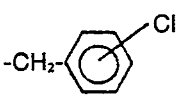 |
| 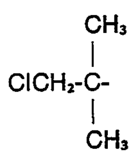 | $CH_3$ | $CH_3$ | H | $-CH_2-$ 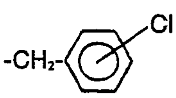 |
| 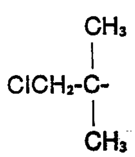 | Cyclohexan | | H | $-CH_2-$ 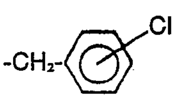 |
| 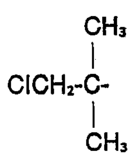 | Cyclohexen | | H | $-CH_2-$ 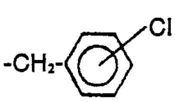 |
| 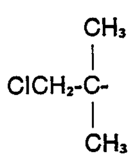 | $CH_3$ | $CH_3$ | $CH_3$ | $-CH_2-$ 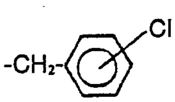 |
| 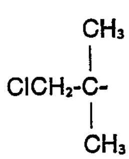 | Cyclohexan | | $CH_3$ | $-CH_2-$ 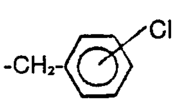 |

| R¹ | R² | R³ | R⁵ | Y |
|---|---|---|---|---|
| 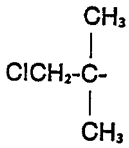 | Cyclohexen | | $CH_3$ | 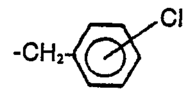 |
| 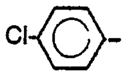 | $CH_3$ | $CH_3$ | H | 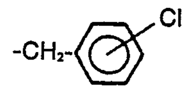 |
| 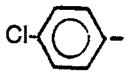 | Cyclohexan | | H | 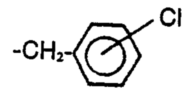 |
| 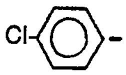 | Cyclohexen | | H | 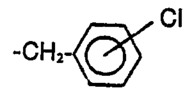 |
| 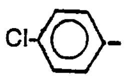 | $CH_3$ | $CH_3$ | $CH_3$ | 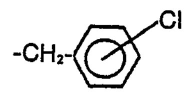 |
| 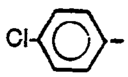 | Cyclohexan | | $CH_3$ | 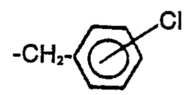 |
| 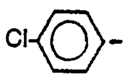 | Cyclohexen | | $CH_3$ | 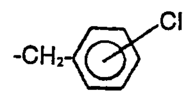 |
| 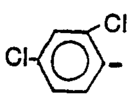 | $CH_3$ | $CH_3$ | H | 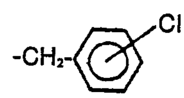 |
| 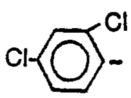 | Cyclohexan | | H | 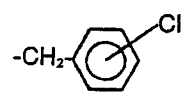 |
| 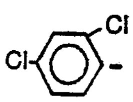 | Cyclohexen | | H | 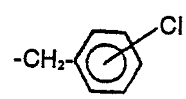 |
| 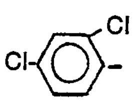 | $CH_3$ | $CH_3$ | $CH_3$ | 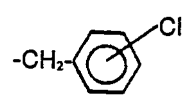 |
| 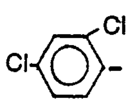 | Cyclohexan | | $CH_3$ | 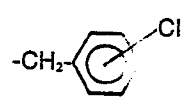 |
| 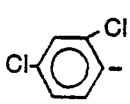 | Cyclohexen | | $CH_3$ | 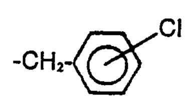 |

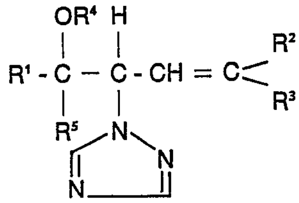

(Ic)

| $R^1$ | $R^2$ | $R^3$ | $R^4$ | $R^5$ |
|---|---|---|---|---|
| $C(CH_3)_3$ | $CH_3$ | $CH_3$ | $C_2H_5$ | H |
| $C(CH_3)_3$ | Cyclohexan | | $C_2H_5$ | H |
| $C(CH_3)_3$ | Cyclohexen | | $C_2H_5$ | H |
| $C(CH_3)_3$ | Methylcyclohexen | | $C_2H_5$ | H |
| $CH_3$<br>$ClCH_2$-C-<br>$CH_3$ | $CH_3$ | $CH_3$ | $C_2H_5$ | $CH_3$ |
| $CH_3$<br>$ClCH_2$-C-<br>$CH_3$ | Cyclohexan | | $C_2H_5$ | $CH_3$ |
| $CH_3$<br>$ClCH_2$-C-<br>$CH_3$ | Cyclohexen | | $C_2H_5$ | $CH_3$ |
| $CH_3$<br>$ClCH_2$-C-<br>$CH_3$ | Methylcyclohexen | | $C_2H_5$ | $CH_3$ |
| $CH_3$<br>$FCH_2$-C-<br>$CH_3$ | $CH_3$ | $CH_3$ | $C_2H_5$ | H |
| $CH_3$<br>$FCH_2$-C-<br>$CH_3$ | Cyclohexan | | $C_2H_5$ | H |
| $CH_3$<br>$FCH_2$-C-<br>$CH_3$ | Cyclohexen | | $C_2H_5$ | H |
| $CH_3$<br>$FCH_2$-C-<br>$CH_3$ | Methylcyclohexen | | $C_2H_5$ | H |

| $R^1$ | $R^2$ | $R^3$ | $R^4$ | $R^5$ |
|---|---|---|---|---|
| 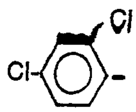 | $CH_3$ | $CH_3$ | $C_2H_5$ | H |
| 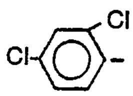 | Cyclohexan | | $C_2H_5$ | H |
| 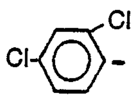 | Cyclohexen | | $C_2H_5$ | H |
| 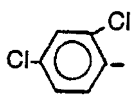 | Methylcyclohexen | | $C_2H_5$ | H |
| $C(CH_3)_3$ | $CH_3$ | $CH_3$ | 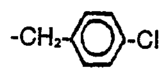 | H |
| $C(CH_3)_3$ | Cyclohexan | | 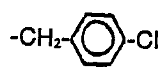 | H |
| $C(CH_3)_3$ | Cyclohexen | | 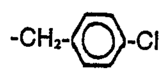 | H |
| $C(CH_3)_3$ | Methylcyclohexen | | 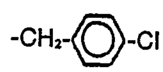 | H |
| 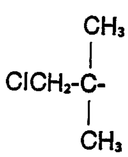 | $CH_3$ | $CH_3$ | 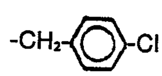 | H |
| 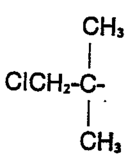 | Cyclohexan | | 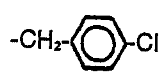 | H |
| 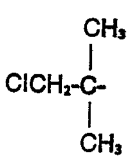 | Cyclohexen | | 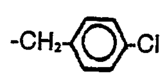 | H |
| 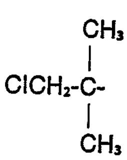 | Methylcyclohexen | | 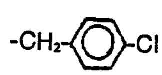 | H |
| 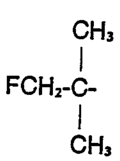 | $CH_3$ | $CH_3$ | 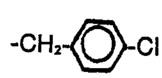 | H |

| $R^1$ | $R^2$ | $R^3$ | $R^4$ | $R^5$ |
|---|---|---|---|---|
| 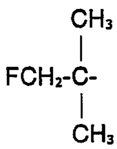 | Cyclohexan | | 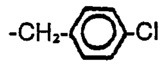 | H |
| 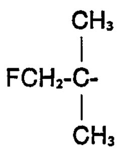 | Cyclohexen | | 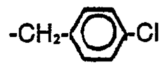 | H |
| 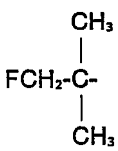 | Methylcyclohexen | | 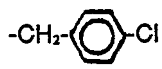 | H |
| 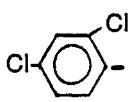 | $CH_3$ | $CH_3$ | 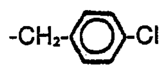 | H |
| 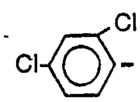 | Cyclohexan | | 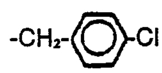 | H |
| 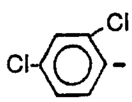 | Cyclohexen | | 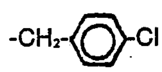 | H |
| 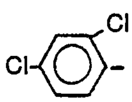 | Methylcyclohexen | | 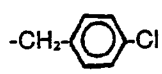 | H |
| 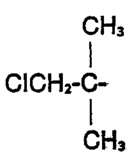 | $CH_3$ | $CH_3$ | $-CO-CH_3-$ | H |
| 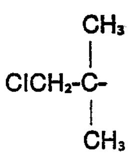 | Cyclohexan | | $-CO-CH_3-$ | H |
| 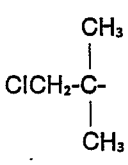 | Cyclohexen | | $-CO-CH_3-$ | H |
| 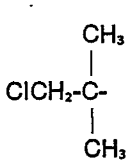 | Methylcyclohexen | | $-CO-CH_3-$ | H |

| $R^1$ | $R^2$ | $R^3$ | $R^4$ | $R^5$ |
|---|---|---|---|---|
| 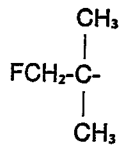 | $CH_3$ | $CH_3$ | $-CO-CH_3-$ | H |
| 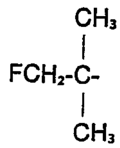 | Cyclohexan | | $-CO-CH_3-$ | H |
| 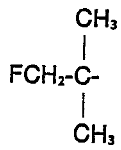 | Cyclohexen | | $-CO-CH_3-$ | H |
| 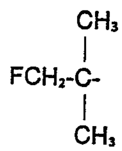 | Methylcyclohexen | | $-CO-CH_3-$ | H |
| 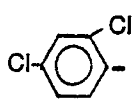 | $CH_3$ | $CH_3$ | $-CO-CH_3-$ | H |
| 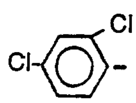 | Cyclohexan | | $-CO-CH_3-$ | H |
| 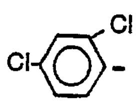 | Cyclohexen | | $-CO-CH_3-$ | H |
| 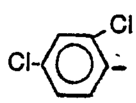 | Methylcyclohexen | | $-CO-CH_3-$ | H |
| 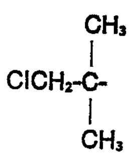 | $CH_3$ | $CH_3$ | $-CO-NHCH_3$ | H |
| 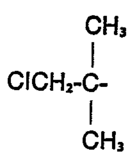 | Cyclohexan | | $-CO-NHCH_3$ | H |
| 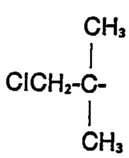 | Cyclohexen | | $-CO-NHCH_3$ | H |

| R¹ | R² | R³ | R⁴ | R⁵ |
|---|---|---|---|---|
| 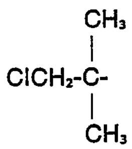 | Methylcyclohexen | | $-CO-NHCH_3$ | H |
| 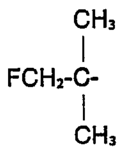 | $CH_3$ | $CH_3$ | $-CO-NHCH_3$ | H |
| 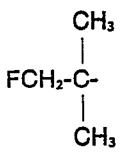 | Cyclohexan | | $-CO-NHCH_3$ | H |
| 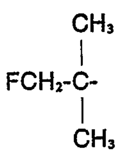 | Cyclohexen | | $-CO-NHCH_3$ | H |
| 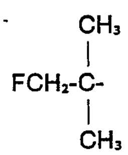 | Methylcyclohexen | | $-CO-NHCH_3$ | H |
| 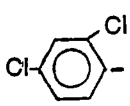 | $CH_3$ | $CH_3$ | $-CO-NHCH_3$ | H |
| 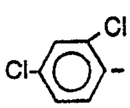 | Cyclohexan | | $-CO-NHCH_3$ | H |
| 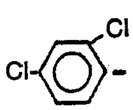 | Cyclohexen | | $-CO-NHCH_3$ | H |
| 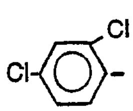 | Methylcyclohexen | | $-CO-NHCH_3$ | H |
| 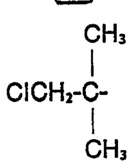 | $CH_3$ | $CH_3$ | -CO-NH-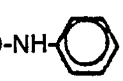 | H |
| 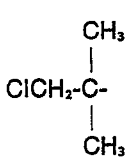 | Cyclohexan | | -CO-NH-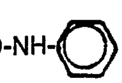 | H |

| $R^1$ | $R^2$ | $R^3$ | $R^4$ | $R^5$ |
|---|---|---|---|---|
| 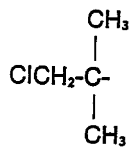 | Cyclohexen | | 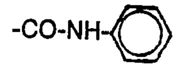 | H |
| 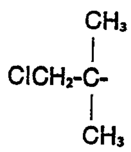 | Methylcyclohexen | | 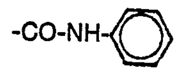 | H |
| 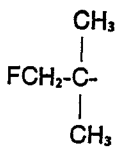 | $CH_3$ | $CH_3$ | 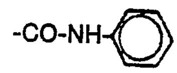 | H |
| 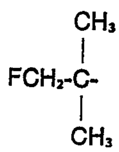 | Cyclohexan | | 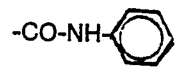 | H |
| 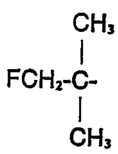 | Cyclohexen | | 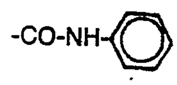 | H |
| 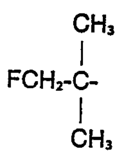 | Methylcyclohexen | | 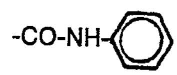 | H |
| 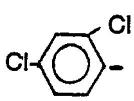 | $CH_3$ | $CH_3$ | 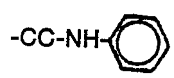 | H |
| 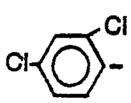 | Cyclohexan | | 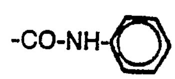 | H |
| 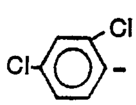 | Cyclohexen | | 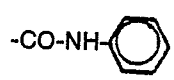 | H |
| 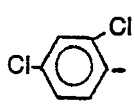 | M[illegible]cyclohexen | | 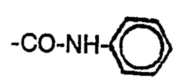 | H |
| 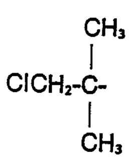 | $CH_3$ | $CH_3$ | 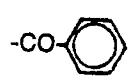 | H |

| $R^1$ | $R^2$ | $R^3$ | $R^4$ | $R^5$ |
|---|---|---|---|---|
| 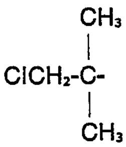 | Cyclohexan | | 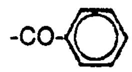 | H |
| 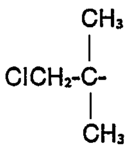 | Cyclohexen | | 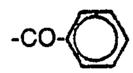 | H |
| 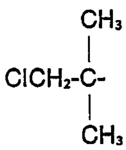 | Methylcyclohexen | | 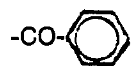 | H |
| 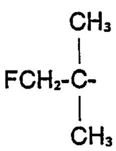 | $CH_3$ | $CH_3$ | 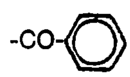 | H |
| 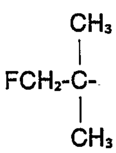 | Cyclohexan | | 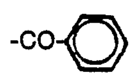 | H |
| 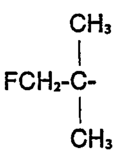 | Cyclohexen | | 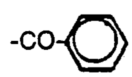 | H |
| 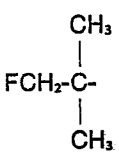 | Methylcyclohexen | | 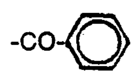 | H |
| 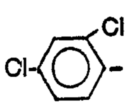 | $CH_3$ | $CH_3$ | 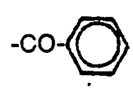 | H |
| 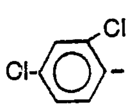 | Cyclohexan | | 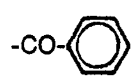 | H |
| 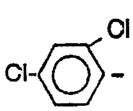 | Cyclohexen | | 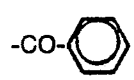 | H |
| 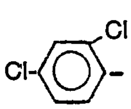 | Methylcyclohexen | | 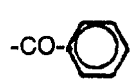 | H |

| $R^1$ | $R^2$ | $R^3$ | $R^4$ | $R^5$ |
|---|---|---|---|---|
| 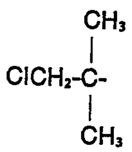 | $CH_3$ | $CH_3$ | $-CO-CHCl_2$ | H |
| 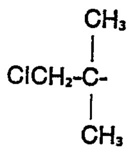 | Cyclohexan | | $-CO-CHCl_2$ | H |
| 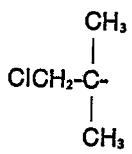 | Cyclohexen | | $-CO-CHCl_2$ | H |
| 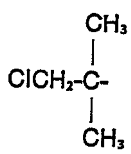 | Methylcyclohexen | | $-CO-CHCl_2$ | H |
| 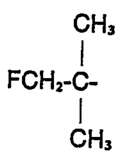 | $CH_3$ | $CH_3$ | $-CO-CHCl_2$ | H |
| 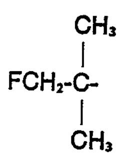 | Cyclohexan | | $-CO-CHCl_2$ | H |
| 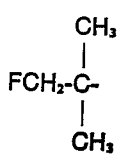 | Cyclohexen | | $-CO-CHCl_2$ | H |
| 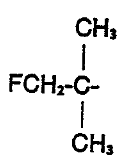 | Methylcyclohexen | | $-CO-CHCl_2$ | H |
| 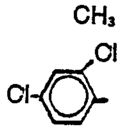 | $CH_3$ | $CH_3$ | $-CO-CHCl_2$ | H |
| 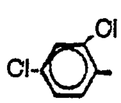 | Cyclohexan | | $-CO-CHCl_2$ | H |
| 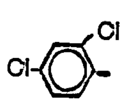 | Cyclohexen | | $-CO-CHCl_2$ | H |

| $R^1$ | $R^2$ | $R^3$ | $R^4$ | $R^5$ |
|---|---|---|---|---|
| 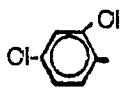 | Methylcyclohexen | | $-CO-CHCl_2$ | H |
| $C(CH_3)_3$ | $CH_3$ | $CH_3$ | $-CO-CH_3$ | H |
| $C(CH_3)_3$ | Cyclohexan | | $-CO-CH_3$ | H |
| $C(CH_3)_3$ | Cyclohexen | | $-CO-CH_3$ | H |
| $C(CH_3)_3$ | Methylcyclohexen | | $-CO-CH_3$ | H |
| $C(CH_3)_3$ | $CH_3$ | $CH_3$ | $-CO-NHCH_3$ | H |
| $C(CH_3)_3$ | Cyclohexan | | $-CO-NHCH_3$ | H |
| $C(CH_3)_3$ | Cyclohexen | | $-CO-NHCH_3$ | H |
| $C(CH_3)_3$ | Methylcyclohexen | | $-CO-NHCH_3$ | H |
| $C(CH_3)_3$ | $CH_3$ | $CH_3$ | -CO-NH- 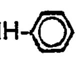 | H |
| $C(CH_3)_3$ | Cyclohexan | | -CO-NH- 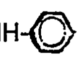 | H |
| $C(CH_3)_3$ | Cyclohexen | | -CO-NH- 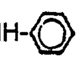 | H |
| $C(CH_3)_3$ | Methylcyclohexen | | -CO-NH- 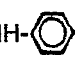 | H |
| $C(CH_3)_3$ | $CH_3$ | $CH_3$ | -CO- 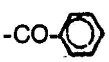 | H |
| $C(CH_3)_3$ | Cyclohexan | | -CO- 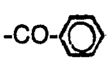 | H |
| $C(CH_3)_3$ | Cyclohexen | | -CO- 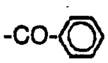 | H |
| $C(CH_3)_3$ | Methylcyclohexen | | -CO- 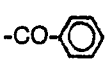 | H |
| $C(CH_3)_3$ | $CH_3$ | $CH_3$ | $-CO-CHCl_2$ | H |
| $C(CH_3)_3$ | Cyclohexan | | $-CO-CHCl_2$ | H |
| $C(CH_3)_3$ | Cyclohexen | | $-CO-CHCl_2$ | H |
| $C(CH_3)_3$ | Methylcyclohexen | | $-CO-CHCl_2$ | H |

Verwendet man beispielsweise Pinakolyl-1,2,4-triazol und Cyclohexancarbaldehyd als Ausgangsstoffe, so kann der Reaktionsablauf durch das folgende Formelschema wiedergegeben werden (Verfahren a):

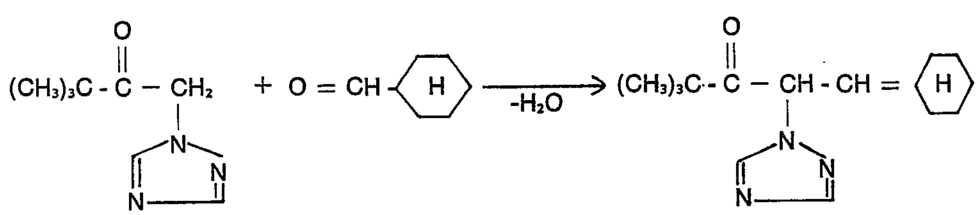

Verwendet man 1-Cyclohexyliden-4,4-dimethyl-2-(1,2,4-triazol-1-yl)-pentan-3-on und Natriumborhydrid als Ausgangsstoffe, so kann der Reaktionsablauf durch das folgende Formelschema wiedergegeben werden (Verfahren b/α):

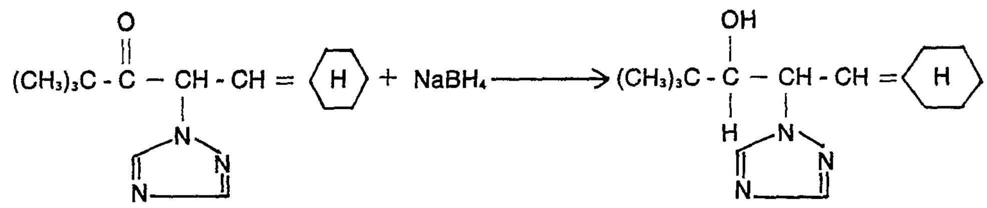


Verwendet man 1-Cyclohexyliden-4,4-dimethyl-2-(1,2,4-triazol-1-yl)-penten-3-on und Methylmagnesiumbromid als Ausgangsstoffe, so kann der Reaktionsablauf durch das folgende Formelschema wiedergegeben werden (Verfahren b/β):

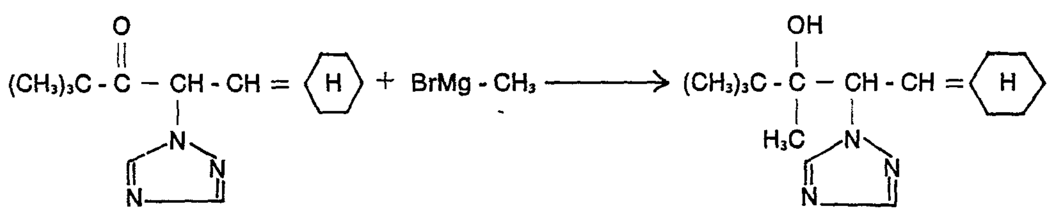


Verwendet man 1-Cyclohexyliden-4,4-dimethyl-1-(1,2,4-triazol-1-yl)-pentan-3-ol, Natriumhydrid und Ethylbromid als Ausgangsstoffe, so kann der Reaktionsablauf durch das folgende Formelschema wiedergegeben werden (Verfahren c):

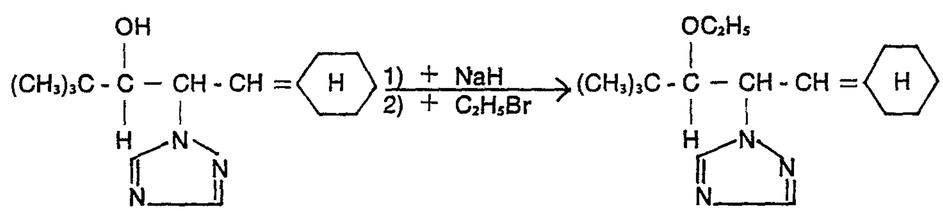


Verwendet man 1-Cyclohexyliden-4,4-dimethyl-2-(1,2,4-triazol-1-yl)-pentan-3-ol, Natriumhydrid und Acetylchlorid als Ausgangsstoffe, so kann der Reaktionsablauf durch das folgende Formelschema wiedergegeben werden (Verfahren c):

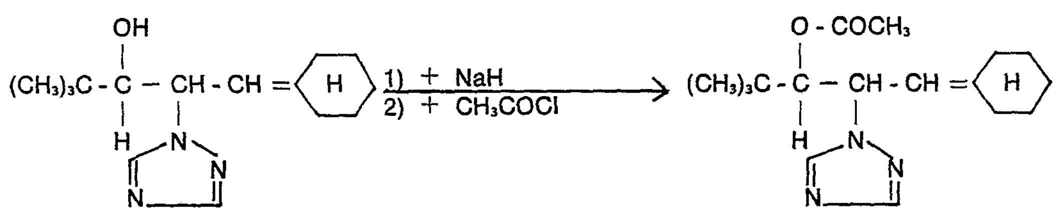


Verwendet man 1-Cyclohexyliden-4,4-dimethyl-2-(1,2,4-triazol-1-yl)-pentan-3-ol und Essigsäureanhydrid als Ausgangstoffe, so kann der Reaktionsablauf durch das folgende Formelschema wiedergegeben werden (Verfahren d):

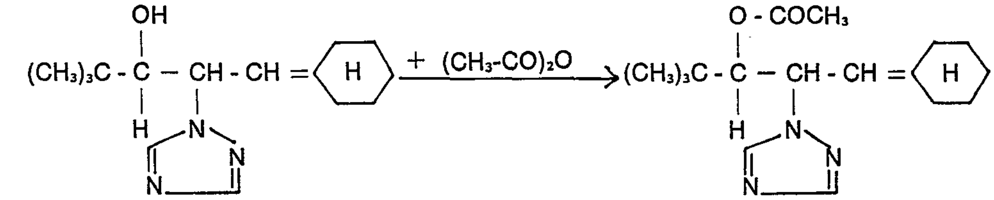


Verwendet man 1-Cyclohexyliden-4,4-dimethyl-2-(1,2,4-triazol-1-yl)-pentan-3-ol und Phenylisocyanat als Ausgangsstoffe, so kann der Reaktionsablauf durch das folgende Formelschema wiedergegeben werden (Verfahren e):

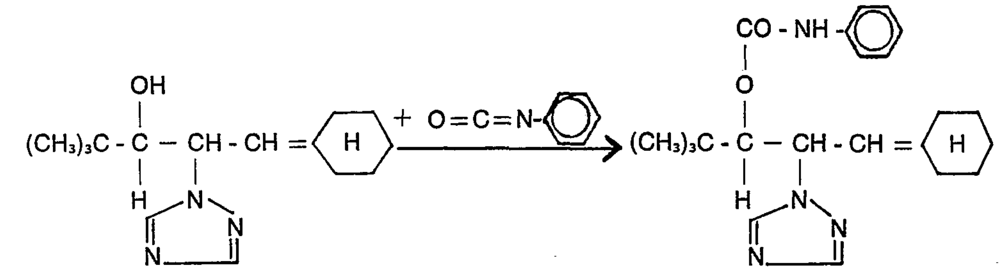


Verwendet man 2,2,6-Trimethyl-4-(1,2,4-triazol-1-yl)-1-hepten-3-on, Natriumhydrid und 2-Chlorbenzylbromid als Ausgangsstoffe, so kann der Reaktionsablauf durch das folgende Formelschema wiedergegeben werden (Verfahren f):

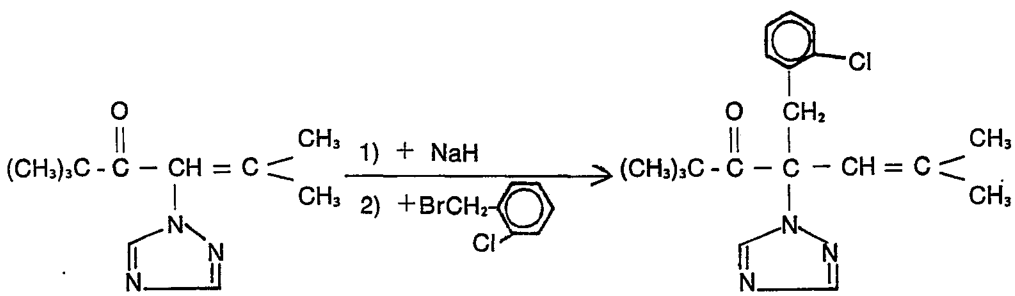


Die bei der Durchführung des erfindungsgemässen Verfahrens (a) als Ausgangsstoffe benötigten Triazol-ketone sind durch die Formel (II) allgemein definiert. In dieser Formel steht $\underline{R^1}$ vorzugsweise für diejenigen Reste, die bereits im Zusammenhang mit der Beschreibung der erfindungsgemässen Stoffe der Formel (I) vorzugsweise für diesen Substituenten genannt wurden.

Die Triazol-ketone der Formel (II) sind weitgehend bekannt (vergleiche DE-OS 2 431 407 [LeA 15 735], DE-OS 2 610 022 und DE-OS 2 638 470). Sie werden erhalten, indem man die entsprechenden Halogen-ketone in Gegenwart eines Säurebinders mit 1,2,4-Triazol umsetzt. Als Beispiele seien die Verbindungen der folgenden Tabelle genannt:

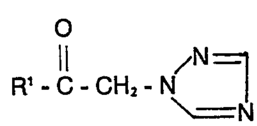
(II)

| $R^1$ | $R^1$ | $R^1$ |
|---|---|---|
| $-C(CH_3)_3$ | $-CH(CH_3)_2$ | $-CH_3$ |
| 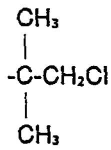 | 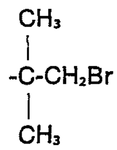 | 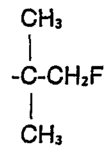 |
| 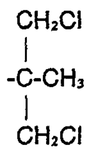 | 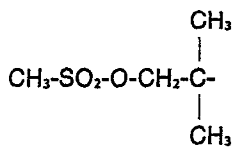 | 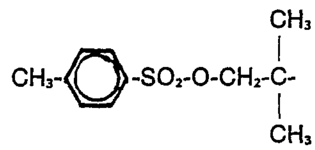 |
| 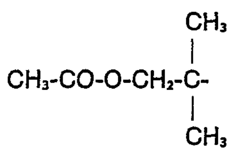 | 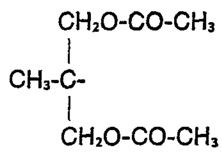 | 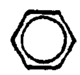 |
| 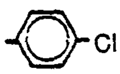 | 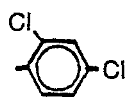 | 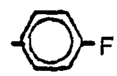 |
| 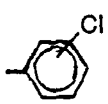 | 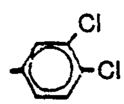 | 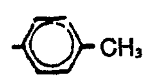 |
| 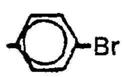 | 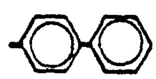 | 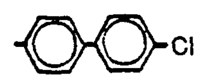 |
| 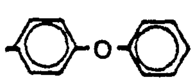 | 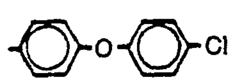 | 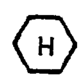 |

Die ausserdem für das erfindungsgemässe Verfahren (a) als Ausgangsstoffe zu verwendenden Aldehyde sind durch die Formel (III) allgemein definiert. In dieser Formel stehen $R^2$ und $R^3$ vorzugsweise für diejenigen Reste, die bereits im Zusammenhang mit der Beschreibung der erfindungsgemässen Stoffe der Formel (I) vorzugsweise für diese Substituenten genannt wurden.

Die Aldehyde der Formel (III) sind allgemein bekannte Verbindungen der organischen Chemie. Als Beispiele seien die folgenden Verbindungen genannt:

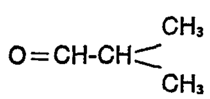 , 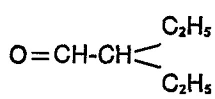 , 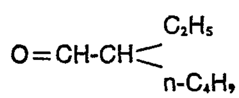 ,

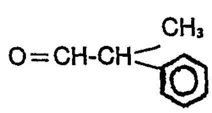 , 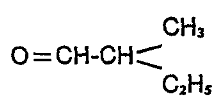 , 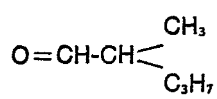 ,

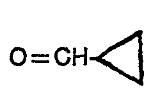 , 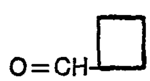 , 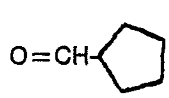 ,

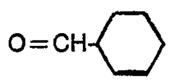 , 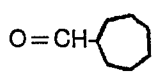 , 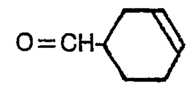 ,

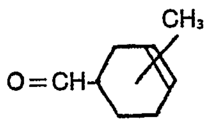 , 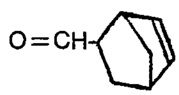 , 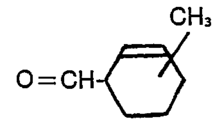 ,

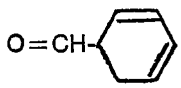 , 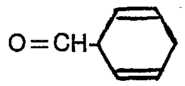 , 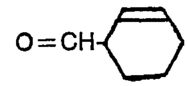 .

Die für Verfahren (b) und (f) als Ausgangsstoffe zu verwendenden Verbindungen sind durch die Formel (Ia) allgemein definiert. In dieser Formel stehen $R^1$, $R^2$ und $R^3$ vorzugsweise für diejenigen Reste, die bereits im Zusammenhang mit der Beschreibung der erfindungsgemässen Stoffe der Formel (I) vorzugsweise für diese Substituenten genannt wurden.

Die ausserdem für das Verfahren (b/α) benötigten komplexen Hydride sind allgemein bekannte Verbindungen der organischen Chemie. Als Beispiele seien vorzugsweise Natriumborhydrid, Natriumcyanoborhydrid und Lithiumalanat genannt.

Die ausserdem für das Verfahren (b/β) als Ausgangsstoffe zu verwendenden Grignard-Verbindungen sind durch die Formel (IV) allgemein definiert. In dieser Formel steht $R^6$ vorzugsweise für diejenigen Reste, die bereits im Zusammenhang mit der Beschreibung der erfindungsgemässen Stoffe der Formel (I) vorzugsweise für $R^5$ in seiner Bedeutung als Alkyl und gegebenenfalls substituiertes Aralkyl genannt wurden. Hal steht vorzugsweise für Chlor, Brom oder Jod.

Die Grignard-Verbindungen der Formel (IV) sind allgemein bekannte Verbindungen der organischen Chemie. Als Beispiele seien genannt: Methylmagnesiumbromid, Ethylmagnesiumbromid, Isopropylmagnesiumbromid und Benzylmagnesiumbromid.

Die für die Verfahren (c), (d) und (e) als Ausgangsstoffe zu verwendenden erfindungsgemässen Stoffe sind durch die Formel (Ib) allgemein definiert. In dieser Formel stehen $R^1$, $R^2$, $R^3$ und $R^5$ vorzugsweise für diejenigen Reste, die bereits im Zusammenhang mit der Beschreibung der erfindungsgemässen Stoffe der Formel (I) vorzugsweise für diese Substituenten genannt sind.

Die ausserdem für das Verfahren (c) als Ausgangsstoffe zu verwendenden Halogenide sind durch die Formel (V) allgemein definiert. In dieser Formel steht $R^7$ vorzugsweise für diejenigen Reste, die bereits im Zusammenhang mit der Beschreibung der erfindungsgemässen Stoffe der Formel (I) vorzugsweise für $R^4$ in seiner Bedeutung als Alkyl, gegebenenfalls substituiertes Aralkyl, Acyl und Carbamoyl genannt wurden. Hal steht vorzugsweise für Fluor, Chlor oder Brom.

Die Halogenide der Formel (V) sind allgemein bekannte Verbindungen der organischen Chemie.

Die ausserdem für das Verfahren (d) als Ausgangsstoffe zu verwendenden Säurehydride sind durch die Formel (VI) allgemein definiert. In dieser Formel steht $R^8$ vorzugsweise für diejenigen Reste, die bereits im Zusammenhang mit der Beschreibung der erfindungsgemässen Stoffe der Formel (I) vorzugsweise für $R^4$ in seiner Bedeutung als Acyl genannt wurden.

Die Säureanhydride der Formel (VI) sind allgemein bekannte Verbindungen der organischen Chemie.

Die ausserdem für das Verfahren (e) als Ausgangsstoffe zu verwendenden Isocyanate sind durch die Formel (VII) allgemein definiert. In dieser Formel steht $R^9$ vorzugsweise für diejenigen Reste, die bereits im Zusammenhang mit der Beschreibung der erfindungsgemässen Stoffe der Formel (I) vorzugsweise für $R^{12}$ in seiner Bedeutung als Alkyl, Halogenalkyl und gegebenenfalls substituiertes Aryl genannt wurden.

Die Isocyanate der Formel (VII) sind allgemein bekannte Verbindungen der organischen Chemie.

Die ausserdem für das Verfahren (f) als Ausgangsstoffe zu verwendenden Halogenide sind durch die Formel (VIII) allgemein definiert. In dieser Formel steht Y vorzugsweise für diejenigen Reste, die bereits im Zusammenhang mit der Beschreibung der erfindungsgemässen Stoffe der Formel (I) vorzugsweise für R in seiner Bedeutung als Alkyl oder gegebenenfalls substituiertes Aralkyl genannt wurden. Hal steht vorzugsweise für Fluor, Chlor oder Brom.

Die Halogenide der Formel (VIII) sind allgemein bekannte Verbindungen der organischen Chemie.

Als Lösungsmittel kommen für das erfindungsgemässe Verfahren (a) vorzugsweise inerte organische Lösungsmittel in Frage. Hierzu gehören vorzugsweise Alkohole, wie Methanol und Ethanol; Ether, wie Tetrahydrofuran und Dioxan; aliphatische und cycloaliphatische Kohlenwasserstoffe, wie Hexan und Cyclohexan; aromatische

Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol und Dichlorbenzol.

Das erfindungsgemässe Verfahren (a) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen, wie Pyridin und Piperidin; sowie insbesondere Piperidinacetat.

Die Reaktionstemperaturen können bei der Durchführung des erfindungsgemässen Verfahrens (a) in einem grösseren Bereich variiert werden. Im allgemeinen arbeitet man zwischen 20 und 160°C, vorzugsweise bei der Siedetemperatur des jeweiligen Lösungsmittels.

Bei der Durchführung des erfindungsgemässen Verfahrens (a) setzt man auf 1 Mol Triazolketon der Formel (II) 1 bis 1,5 Mol Aldehyd der Formel (III) und katalytische bis 0,2 molare Mengen an Katalysator ein. Zur Isolierung der Verbindungen der Formel (I) wird das gewünschte Produkt nach üblichen Methoden, wie z.B. durch Salzbildung (vergleiche die Herstellungsbeispiele) oder chromatographische Aufarbeitung isoliert. Eine eindeutige Charakterisierung erfolgt aufgrund spektroskopischer Daten, wie insbesondere der NMR-Spektren.

Als Lösungsmittel kommen für das erfindungsgemässe Verfahren (b/α) vorzugsweise polare organische Lösungsmittel in Frage. Hierzu gehören vorzugsweise Alkohole, wie Methanol, Ethanol, Isopropanol oder Butanol; und Ether, wie Diethylether oder Tetrahydrofuran; sowie gegebenenfalls deren wässrige Lösungen.

Die Reaktionstemperaturen können bei der Durchführung des erfindungsgemässen Verfahrens (b/α) in einem grösseren Bereich variiert werden. Im allgemeinen arbeitet man zwischen 0 bis 30°C, vorzugsweise bei 0 bis 20°C.

Bei der Durchführung des erfindungsgemässen Verfahrens (b/α) arbeitet man vorzugsweise in molaren Mengen oder einem Überschuss an Reduktionsmittel. Zur Isolierung der Verbindungen der Formel (I) wird das Reaktionsgemisch in verdünnter Salzsäure aufgenommen und mit einem organischen Lösungsmittel extrahiert. Die weitere Aufarbeitung erfolgt in üblicher Weise.

Als Lösungsmittel kommen für das erfindungsgemässe Verfahren (b/β) vorzugsweise wasserfreie Ether, wei Diethylether, Dibutylether und Tetrahydrofuran in Frage.

Die Reaktionstemperaturen können bei der Durchführung des erfindungsgemässen Verfahrens (b/β) in einem grösseren Bereich variiert werden. Im allgemeinen arbeitet man zwischen 0 und 80°C, vorzugsweise zwischen 30 und 60°C.

Bei der Durchführung des erfindungsgemässen Verfahrens (b/β) setzt man auf 1 Mol der Verbindung der Formel (Ia) 2 bis 3 Mol Grignard/Verbindung ein. Die Isolierung der Verbindungen der Formel (I) erfolgt in üblicher und allgemein bekannter Weise.

Als Lösungsmittel kommen für das erfindungsgemässe Verfahren (c) vorzugsweise inerte organische Lösungsmittel in Frage. Hierzu gehören vorzugsweise Ether, wie Diethylether und Dioxan; aromatische Kohlenwasserstoffe, wie Toluol und Benzol; in einzelnen Fällen auch chlorierte Kohlenwasserstoffe, wie Chloroform, Methylenchlorid oder Tetrachlorkohlenstoff; sowie Ketone, wie Aceton oder Methylethylketon; und Nitrile, wie Acetonitril. Der Einfachheit halber kann gegebenenfalls auch ein eingesetztes Säurehalogenid als Lösungsmittel verwendet werden, womit ein entsprechender Überschuss erforderlich wird.

Die Reaktionstemperaturen können bei der Durchführung des erfindungsgemässen Verfahrens (c) in einem grösseren Bereich variiert werden. Im allgemeinen arbeitet man zwischen 20 und 150°C, vorzugsweise zwischen 20 und 100°C, bzw. bei der Siedetemperatur des jeweiligen Lösungsmittels.

Das erfindungsgemässe Verfahren (c) kann gegebenenfalls in Gegenwart einer starken Base durchgeführt werden. Hierzu gehören vorzugsweise Alkalimetall-hydride, Alkalimetall-amide und Alkalimetall-alkoholate, wie z.B. Natriumhydrid, Natriumamid und Kalium-tert.-butylat.

Das erfindungsgemässe Verfahren (c) kann gegebenenfalls in Gegenwart von Säurebindern (Halogenwasserstoff-Akzeptoren) durchgeführt werden. Hierzu gehören organische Basen, vorzugsweise tertiäre Amine, wie z.B. Triethylamin; ferner anorganische Basen, wie z.B. Alkalihydroxide und Alkylicarbonate.

Bei der Durchführung des erfindungsgemässen Verfahrens (c) setzt man vorzugsweise auf 1 Mol der Verbindungen der Formel (Ib) 1 bis 3 Mol Halogenid der Formel (V) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

In einer bevorzugten Ausführungsform wird zweckmässigerweise so verfahren, dass man von einer Verbindung der Formel (Ib) ausgeht, letztere in einem geeigneten inerten organischen Lösungsmittel mittels Alkalimetall-hydrid oder Alkalimetall-amid in das Alkenolat überführt, und letzteres ohne Isolierung sofort mit einem Halogenid der Formel (V) umsetzt, wobei unter Austritt von Alkalihalogenid die erfindungsgemässen Verbindungen der Formel (I) in einem Arbeitsgang erhalten werden.

Nach einer weiteren bevorzugten Ausführungsform wird die o.g. bevorzugte Ausführung bei der Umsetzung von Halogeniden der Formel (V), in welchen $R^7$ für Alkyl oder gegebenenfalls substituiertes Aralkyl steht, in einem Zweiphasensystem vorgenommen, wie beispielsweise wässriges Natron- oder Kalilauge/Toluol oder Methylenchlorid, unter Zusatz von 0,01 - 1 Mol

eines Phasen-Transfer-Katalysators, wie beispielsweise Ammonium- oder Phosphoniumverbindungen, beispielsweise seien Benzyl-dodecyl-dimethyl-ammoniumchlorid und Triethyl-benzyl-ammoniumchlorid genannt.

Als Lösungsmittel kommen für das erfindungsgemässe Verfahren (d) vorzugsweise inerte organische Lösungsmittel in Frage. Hierzu gehören vorzugsweise die beim Verfahren (c) aufgezählten Solventien sowie die jeweils verwendeten Säureanhydride der Formel (VI).

Als Katalysatoren können beim Verfahren (d) vorzugsweise alle üblichen sauren und basischen Katalysatoren verwendet werden, wie z.B. Schwefelsäure, Chlorwasserstoff, Bromwasserstoff, Bortrifluorid, Zinkchlorid, Natriumacetat, Natriumbenzoat, Natriumcarbonat, Calciumoxid, Magnesiumoxid, Pyridin und Triethylamin.

Die Reaktionstemperaturen können bei der Durchführung des erfindungsgemässen Verfahrens (d) in einem grösseren Bereich variiert werden. Im allgemeinen arbeitet man zwischen 20 und 150°C, vorzugsweise zwischen 50 und 120°C.

Bei der Durchführung des Verfahrens (d) arbeitet man vorzugsweise in molaren Mengen. Der Einfachheit halber kann man das eingesetzte Säureanhydrid der Formel (VI) auch als Lösungsmittel verwenden, womit ein entsprechender Überschuss erforderlich wird. Die Isolierung der Verbindungen der Formel (I) erfolgt in üblicher Weise.

Als Lösungsmittel kommen für das erfindungsgemässe Verfahren (e) vorzugsweise inerte organische Lösungsmittel in Frage. Hierzu gehören vorzugsweise die beim Verfahren (c) aufgeführten Solventien.

Als Katalysatoren können beim Verfahren (e) vorzugsweise verwendet werden: tertiäre Basen, wie Triethylamin und Pyridin-, oder Zinn-organische Verbindungen, wie Dibutylzinn-dilaurat und Tributylzinn-laurat.

Die Reaktionstemperaturen können bei der Durchführung des erfindungsgemässen Verfahrens (e) in einem grösseren Bereich variiert werden. Im allgemeinen arbeitet man zwischen 0 und 100°C, vorzugsweise zwischen 20 und 40°C.

Bei der Durchführung des erfindungsgemässen Verfahrens (e) arbeitet man vorzugsweise in molaren Mengen. Zur Isolierung der Verbindungen der Formel (I) wird das Lösungsmittel abdestilliert und der Rückstand nach üblichen Methoden aufgearbeitet.

Als Lösungsmittel für das erfindungsgemässe Verfahren (f) kommen vorzugsweise inerte organische Lösungsmittel in Frage. Hierzu gehören vorzugsweise Ether, wie Diethylether und Dioxan; aromatische Kohlenwasserstoffe, wie Toluol und Benzol; sowie insbesondere Dimethylformamid.

Die Reaktionstemperaturen können bei der Durchführung des Verfahrens (f) in einem grösseren Bereich variiert werden. Im allgemeinen arbeitet man zwischen 20 und 150°C, vorzugsweise zwischen 20 und 100°C.

Das erfindungsgemässe Verfahren (f) wird in Gegenwart einer starken Base durchgeführt. Hierzu gehören vorzugsweise Alkalimetallhydride und Alkalimetall-amide, wie z.B. Natriumhydrid und Natriumamid.

Bei der Durchführung des erfindungsgemässen Verfahrens (f) setzt man vorzugsweise auf 1 Mol der Verbindungen der Formel (Ia) 1 bis 3 Mol Halogenid der Formel (VIII) ein. Die Isolierung der Verbindungen der Formel (I) erfolgt in üblicher und allgemein bekannter Weise.

Die nach den Verfahren (a) bis (f) herstellbaren erfindungsgemässen Stoffe der Formel (I) können in Säureadditions-Salze bzw. Metallsalz-Komplexe überführt werden.

Zur Herstellung von physiologisch verträglichen Säureadditionssalzen der Verbindungen der Formel (I) kommen vorzugsweise folgende Säuren in Frage: Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäure, wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmitttel gereinigt werden.

Zur Herstellung von Metallsalz-Komplexen der Verbindungen der Formel (I) kommen vorzugsweise Salze von Metallen der II. bis IV. Hauptgruppe und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metall-Komplexe von Verbindungen der Formel (I) können in einfacher Wiese nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisieren reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäss verwendbaren Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gersten- bzw. des Getreidemehltaus (Erysiphe graminis).

Besonders hervorzuheben ist, dass die erfindungsgemässen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die erfindungsgemäss verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Strassenrändern, auf Flughäfen oder in Obstanlagen reduziert werden. Von Bedeutung ist auch die Hemmung des Wuchses von krautigen und holzigen Pflanzen an Strassenrändern und in der Nähe von Pipeline oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträge bezogen auf die Bodenfläche erzielt werden können. Ein Vorteil der so erzielten kleineren Pflanzen ist auch, dass die Kultur leichter bearbeitet und beerntet werden kann.

Eine Hemmung des vegetativen Wachstums der Pflanzen kann auch dadurch zu Ertragsteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, dadurch dass mehr Assimilate gebildet werden, so dass mehr oder grössere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Ausserdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine grosse Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. grosses Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine grosse Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden («Ausdünnung»), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dass z.B. bei Tabak, Tomaten oder Kaffee ein vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schliesslich kann mit Wachstumsregulatoren eine Resistenz der Pflanzen gegen Frost, Trokkenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure Ester, Polyoxyäthylen-Fettalkohol-Äther, z.B. Alkylarylpolyglykoläther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer,

Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Bei der Anwendung als Pflanzenwachstumsregulatoren können die Wirkstoffkonzentrationen in einem grösseren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, dass die Anwendung der Wachstumsregulatoren in einem bevorzugten Zeitraum vorgenommen wird, dessen genaue Abgrenzung sich nach den klimatischen und vegetativen Gegebenheiten richtet.

Bei der Prüfung auf fungizide Wirksamkeit werden in den nachfolgenden Beispielen die nachstehend angegebenen Verbindungen als Vergleichssubstanzen eingesetzt:

A = 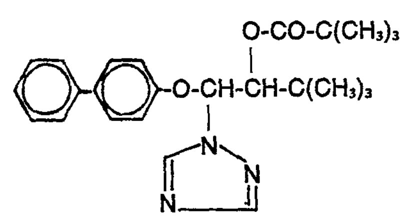

B = 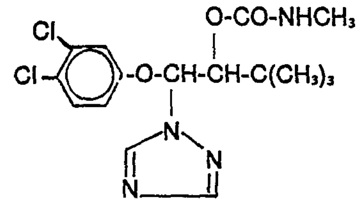

C = 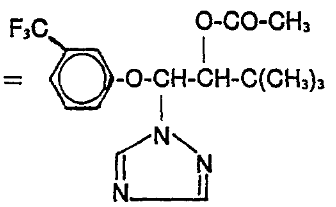

D = 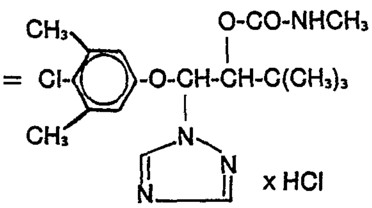
x HCl

E = 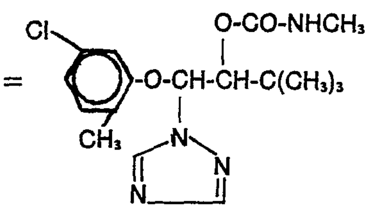

F = 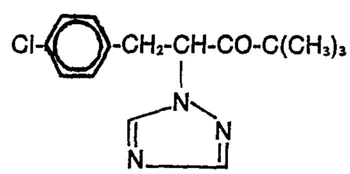

Beispiel A
Sprossbehandlungs-Test/Getreidemehltau/protektiv
(blattzerstörende Mykose)

Zur Herstellung einer zweckmässigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl--polyglykol-ether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gesternjungpflanzen der Sorte Amsel mit der Wirkstoffzubereitung taufeucht. Nach Antrocknen bestäubt man die Gestenpflanzen mit Sporen von Erysiphe graminis var. hordei.

Nach 6 Tagen Verweilzeit der Pflanzen bei einer Temperatur von 21 - 22°C und einer Luftfeuchtigkeit von 80 - 90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Mehltaubefall ist.

Bei diesem Test zeigen z.B. die folgenden Verbindungen eine sehr gute Wirkung, die derjenigen der aus dem Stand der Technik bekannten Verbindungen (A), (B) und (C) überlegen ist: Verbindungen gemäss Herstellungsbeispielen 13, 14, 15, 4, 17, 2, 24, 23, 18, 19, 20, 21, 7 und 3.

Beispiel B
Gerstenmehltau-Test (Erysiphe graminis var. hordei) / systemisch
(pilzliche Getreidesprosskrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulvrigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3x12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21-22°C und 80-90% rel. Luftfeuchte und 16stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Bei diesem Test zeigen z.B. folgende Verbindungen eine sehr gute Wirkung, die derjenigen der aus dem Stand der Technik bekannten Verbindungen (B), (D) und (E) überlegen ist: Verbindungen gemäss Herstellungsbeispielen 13, 15, 2, 8, 24, 18, 5 und 3.

Beispiel C
Myzelwachstums-Test

Verwendeter Nährboden:

| | | |
|---|---|---|
| 20 | Gewichtsteile | Agar-Agar |
| 200 | Gewichtsteile | Kartoffeldekokt |
| 5 | Gewichtsteile | Malz |
| 15 | Gewichtsteile | Dextrose |
| 5 | Gewichtsteile | Pepton |
| 2 | Gewichtsteile | $Na_2HPO_4$ |
| 0,3 | Gewichtsteile | $Ca(NO_3)_2$ |

Verhältnis von Lösungsmittelgemisch zum Nährboden:

| | | |
|---|---|---|
| 2 | Gewichtsteile | Lösungsmittelgemisch |
| 100 | Gewichtsteile | Agarnährboden |

Zusammensetzung Lösungsmittelgemisch

| | | |
|---|---|---|
| 0,19 | Gewichtsteile | DMF oder Aceton |
| 0,01 | Gewichtsteile | Emulgator Alkylarylpolyglykolether |
| 1,80 | Gewichtsteile | Wasser |
| 2 | Gewichtsteile | Lösungsmittelgemisch |

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21°C inkubiert.

Die Auswertung erfolgt je nach Wachstumsgeschwindigkeit der Pilze nach 4-10 Tagen. Bei der Auswertung wird das radiale Myzelwachstum auf den behandelten Nährboden mit dem Wachstum auf dem Kontrollnährboden verglichen. Die Bonitierung des Pilzwachstums geschieht mit folgenden Kennzahlen:

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Bei diesem Test zeigen z.B. die folgenden Verbindungen eine sehr gute Wirkung, die derjenigen der aus dem Stand der Technik bekannten Verbindung (F) überlegen ist: Verbindungen gemäss Herstellungsbeispielen 18, 7, 13, 14 und 2.

Bei der Prüfung auf das Pflanzenwachstum regulierende Wirksamkeit werden in den nachfolgenden Beispielen die nachstehend angegebenen Verbindungen als Vergleichssubstanzen eingesetzt:

A = 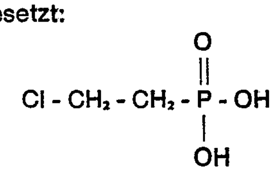

2-Chlorethylphosphonsäure

$$B = Cl-CH_2-CH_2-\overset{\oplus}{N}(CH_3)_3\ Cl^{\ominus}$$

2-Chlorethyl-trimethylammoniumchlorid

Beispiel D
Wuchsbeeinflussung bei Zuckerformamid

Lösungsmittel: 30 Gewichtsteile Dimethylformamid
Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnass mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0% Wuchsbeeinflussung ein Wachstum entsprechend dem der Kontrollpflanze. Negative Werte kennzeichnen eine Wuchshemmung, positive Werte eine Wuchsförderung gegenüber den Kontrollpflanzen.

Die erfindungsgemässen Wirkstoffe 4, 17, 20, 21 und 23 zeigen in diesem Test eine bessere Wuchsbeeinflussung als die aus dem Stand der Technik bekannte Substanz (B).

Beispiel E

Wuchshemmung bei Sojabohnen

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 2, 4, 15, 17, 20 und 21 zeigen in diesem Test gegenüber der Kontrolle eine starke Wachstumshemmung.

Beispiel F

Wuchshemmung bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 2, 14, 17 und 21 zeigen in diesem Test gegenüber der Kontrolle eine starke Wuchshemmung.

Beispiel G

Wuchshemmung bei Gerste

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstenpflanzen werden im Gewächshaus bis zum 2-Blattstadium angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend der Kontrollpflanzen.

Die erfindungsgemässen Wirkstoffe 2 und 21 zeigen in diesem Test eine bessere Wuchshemmung als die aus dem Stand der Technik bekannte Substanz (A).

Herstellungsbeispiele

Beispiel 1

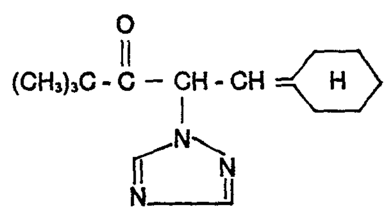

(Verfahren a)

83,5 g (0,5 Mol) Pinakolyltriazol, 60 g (0,54 Mol) Cyclohexancarbaldehyd, 4,2 g (0,05 Mol) Piperidin und 6 g (0,1 Mol) Eisessig in 300 ml Toluol wurden am Wasserabscheider unter Rückfluss erhitzt, bis kein Wasser mehr abgeschieden wird. Die erkaltete Reaktionslösung wird mit gesättigter Natriumchlorid-Lösung gewaschen, getrocknet, filtriert und am Rotationsverdampfer eingeengt.

Der Rückstand wird in 500 ml Aceton aufgenommen und unter Rühren mit einer filtrierten Lösung von 90 g (0,25 Mol) Naphthalin-1,5-disulfonsäure versetzt.

Der dabei ausgefallene Niederschlag wird abgesaugt, mit Aceton gewaschen und in 500 ml Methylenchlorid suspendiert. Danach wird bis

zur alkalischen Reaktion halbkonzentrierte Natriumcarbonatlösung zugegeben, die organische Phase abgetrennt, mit Wasser gewaschen, getrocknet, filtriert und am Rotationsverdampfer eingeengt.

Man erhält 49 g (38,0% der Theorie) 1-Cyclohexyliden-4,4-dimethyl-2-(1,2,4-triazol-1-yl)-pentan-3-on als gelbliches Öl vom Brechungsindex $n_D^{20}$: 1.4990.

Herstellung des Ausgangsproduktes

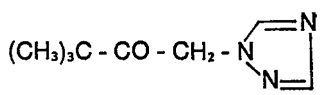

138 g (2 Mol) 1,2,4-Triazol werden bei Raumtemperatur portionsweise zu 276,4 g (2 Mol) gemahlenem Kaliumcarbonat und 269,2 g (2 Mol) α-Chlorpinakolin in 500 ml Aceton gegeben, wobei die Innentemperatur bis zur Siedehitze ansteigt. Man lässt 5 Stunden unter Rückfluss rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72% der Theorie) 3,3-Dimethyl-1-(1,2,4-triazol-1-yl)-butan-2-on vom Schmelzpunkt 62 bis 64°C.

Beispiel 2

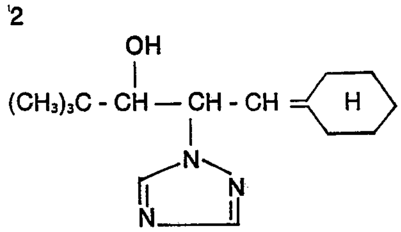

(Verfahren b/α)

52 g (0,2 Mol) 1-Cyclohexyliden-4,4-dimethyl-2-(1,2,4-triazol-1-yl)-pentan-3-on (Beispiel 1) werden in 300 ml Methanol gelöst und unter Rühren und Kühlen portionsweise mit 8,5 g Natriumborhydrid versetzt. Nach beendeter Reaktion wird die Lösung auf pH 6 gebracht und am Rotationsverdampfer eingeengt. Der Rückstand wird in 200 ml Methylenchlorid aufgenommen, mit gesättigter Natriumhydrogencarbonat-Lösung gewaschen, getrocknet, filtriert und am Rotationsverdampfer eingeengt. Der ölige Rückstand wird über die Säule (Chloroform: Methanol = 2 : 1) gereinigt.

Man erhält 33,7 g (64% der Theorie) 1-Cyclohexyliden-4,4-dimethyl-2-(1,2,4-triazol-1-yl)-pentan-3-ol als farbloses Öl vom Brechungsindex $n_D^{20}$: 1.4993.

Beispiel 3

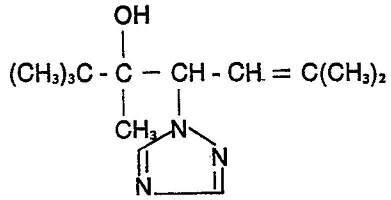

(Verfahren b/β)

Eine aus 69 g (0,5 Mol) Methyljodid, 10 g (0,42 Mol) Magnesium in 150 ml Ether hergestellte Lösung von Methylmagnesiumjodid wird zu einer Lösung von 44 g (0,2 Mol) 2,2,6-Trimethyl-4-(1,2,4-triazol-1-yl)-5-hepten-3-on (Herstellung gemäss Beispiel 1) in 150 ml Ether langsam unter Kühlung getropft und anschliessend 1 Stunde unter Rückfluss erhitzt. Das Reaktionsgemisch wird auf eine wässrige Ammoniumchlorid-Lösung gegossen und die Etherphase abgetrennt. Die wässrige Phase wird nochmals mit Ether extrahiert, die vereinigten Etherphasen mit Wasser gewaschen, über Natriumsulfat getrocknet und das Lösungsmittel abgezogen.

Der Rückstand wird mit 0,05 Mol Naphthalin-1,5-disulfonsäure in 100 ml Aceton versetzt und der ausgefallene Niederschlag abgesaugt (23,1 g). Der Niederschlag wird in 200 ml Wasser suspendiert und 8,4 g (0,1 Mol) Natriumhydrogencarbonat zugegeben und der Niederschlag abgesaugt.

Der Feststoff wird aus Cyclohexan umkristallisiert. Man erhält 11,2 g (23,2% der Theorie) 2,2,3,6-Tetramethyl-4-(1,2,4-triazol-1-yl)-5-hepten-3-ol vom Schmelzpunkt 115-116°C.

Beispiel 4

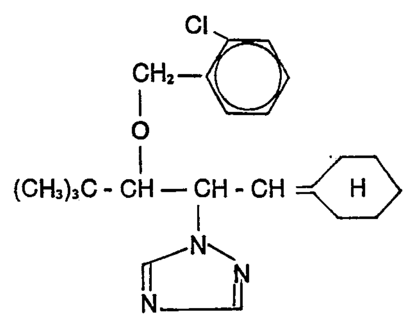

(Verfahren c)

Eine Lösung von 13,1 g (0,15 Mol) 1-Cyclohexyliden-4,4-dimethyl-2-(1,2,4-triazol-1-yl)-pentan-3-ol (Beispiel 2) in 50 ml Dioxan werden zu einer Suspension von 2,0 g 80%igem Natriumhydrid in 50 ml Dioxan getropft und anschliessend 45 Minuten auf 65°C erwärmt. Nach dem Abkühlen werden 10,0 g (0,06 Mol) 2-Chlorbenzylchlorid eingetropft und das Gemisch über Nacht unter Rückfluss erhitzt. Danach werden 5 ml Methanol zugegeben und das Reaktionsgemisch eingeengt. Der Rückstand wird in Methylenchlorid aufgenommen und mehrmals mit Wasser gewaschen. Die organische Phase wird über Natriumsulfat getrocknet, eingeengt und schliesslich im Hochvakuum entgast. Man erhält 14,2 g (72% der Theorie) 1-Cyclohexyliden-3-(2-chlorbenzyloxy)-4,4-dimethyl-2-(1,2,4-triazol-1-yl)-pentan als gelbliches Öl mit einem Brechungsindex von $n_D^{20}$ = 1.5349.

Beispiel 5

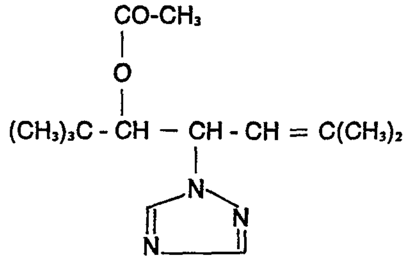

(Verfahren d)

Zu einer Lösung von 11,0 g (0,05 Mol) 2,2,6-Trimethyl-4-(1,2,4-triazol-1-yl)-5-hepten-3-ol (Herstellung gemäss Beispiel 2) in 100 ml Acetanhydrid werden 2 ml Pyridin gegeben und das Gemisch vier Stunden bei 70°C gerührt. Danach wird das Reaktionsgemisch auf Wasser gegossen und mit Natriumhydrogencarbonat neutralisiert. Die wässrige Phase wird mehrmals mit Ether extrahiert, die Etherphase über Natriumsulfat getrocknet und eingeengt. Man erhält 9,7 g (74% der Theorie) 3-Acetoxy-2,2,6-trimethyl-4-(1,2,4-triazol-1-yl)-5-hepten als farbloses Öl mit einem Brechungsindex von $n_D^{20}$ = 1.4809.

Beispiel 6

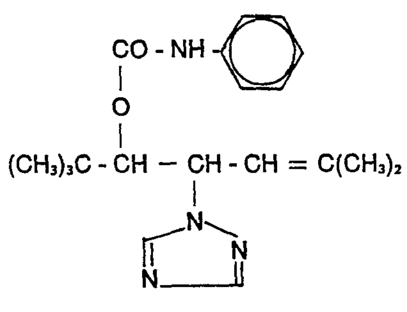

(Verfahren e)

Zu einer Lösung von 15,0 g (0,07 Mol) 2,2,6-Trimethyl-4-(1,2,4-triazol-1-yl)-5-hepten-3-ol (Herstellung gemäss Beispiel 2) in 50 ml Methylenchlorid werden 8,33 g (0,07 Mol) Phenylisocyanat und 2 Tropfen Desmorapid gegeben und das Gemisch 5 Stunden unter Rückfluss erhitzt. Nach Abziehen des Lösungsmittels wird der Rückstand mit Ether verrührt und der ausgefallene Niederschlag abgesaugt. Man erhält 3,0 g (38% der Theorie) 3-Phenylcarbamoyloxy-2,2,6-trimethyl-4-(1,2,4-triazol-1-yl)-5-hepten vom Schmelzpunkt 185-187°C.

Beispiel 7

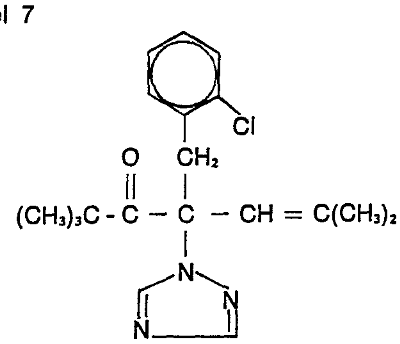

(Verfahren f)

Zu einer Suspension von 6 g Natriumhydrid in 60 ml Dimethylformamid werden 44 g (0,2 Mol) 2,2,6-Trimethyl-4-(1,2,4-triazol-1-yl)-5-hepten-3-on (Herstellung gemäss Beispiel 1), gelöst in 40 ml Dimethylformamid zugetropft und danach 1 Stunde bei Raumtemperatur nachgerührt. Dann werden 32,2 g (0,2 Mol) 2-Chlorbenzylchlorid zugetropft (leicht exotherm) und das Reaktionsgemisch über Nacht bei Raumtemperatur gerührt. Danach wird die Lösung auf Wasser gegossen und mit Essigsäure angesäuert. Die wässrige Phase wird mehrmals mit je 50 ml Methylenchlorid extrahiert, die organische Phase mit Wasser gewaschen, über Natriumsulfat getrocknet und durch Abdestillieren des Lösungsmittels eingeengt. Man erhält 28 g (40% der Theorie) 4-(2-Chlorbenzyl)-2,2,6-trimethyl-4-(1,2,4-trizalo-1-yl)-5-hepten-3-on vom Schmelzpunkt 115-116°C.

In entsprechender Weise werden die Verbindungen der allgemeinen Formel

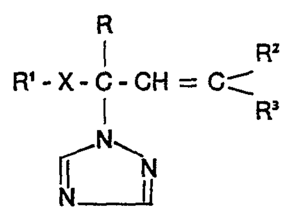

(I)

erhalten:

| Beispiel Nr. | R¹ | X | R | R² | R³ | Physikalische Konstante |
|---|---|---|---|---|---|---|
| 8 | $(CH_3)_3C$ | -CO- | H | $CH_3$ | $CH_3$ | Kp: 98-100°C /0,1 mmHg |
| 9 | $(CH_3)_3C$ | -CO- | H | 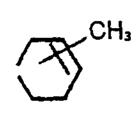 | | Öl |
| 10 | $(CH_3)_3C$ | -CO- | H | 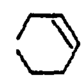 | | Fp: 42°C |

| Beispiel Nr. | $R^1$ | X | R | $R^2$ | $R^3$ | Physikalische Konstante |
|---|---|---|---|---|---|---|
| 11 | $(CH_3)_3C$ | -CO- | H | $C_2H_5$ | $C_2H_5$ | Kp: 105°C /0,1 mmHg |
| 12 | $(CH_3)_3C$ | -CO- | 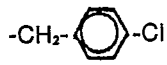 | $CH_3$ | $CH_3$ | Öl, $n_D^{20}$: 1.5424 |
| 13 | $(CH_3)_3C$ | -CH(OH)- | H | | 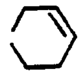 | Öl, $n_D^{20}$: 1.5210 |
| 14 | $(CH_3)_3C$ | -CH(OH)- | H | | 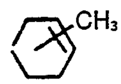 | Fp: 138°C ($xH_2SO_4$) |
| 15 | $(CH_3)_3C$ | -CH(OH)- | H | | 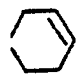 | Fp: 153°C ($xH_2SO_4$) |
| 16 | $(CH_3)_3C$ | -CH(OH)- | 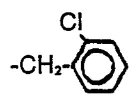 | $CH_3$ | $CH_3$ | Öl, $n_D^{20}$: 1.5394 |
| 17 | $(CH_3)_3C$ | 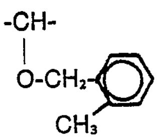 | H | | 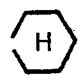 | Öl, $n_D^{20}$: 1.5304 |
| 18 | $(CH_3)_3C$ | 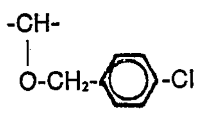 | H | $CH_3$ | $CH_3$ | Fp: 150°C |
| 19 | $(CH_3)_3C$ | 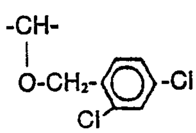 | H | $CH_3$ | $CH_3$ | Kp: 150°C /0,1 mmHg |
| 20 | $(CH_3)_3C$ | 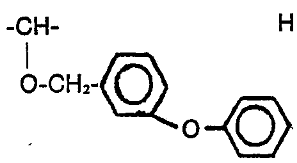 | H | $CH_3$ | $CH_3$ | Kp: 150°C /0,1 mmHg |
| 21 | $(CH_3)_3C$ | 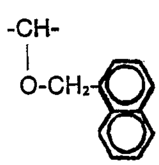 | H | $CH_3$ | $CH_3$ | Kp: 140°C /0,1 mmHg |
| 22 | $(CH_3)_3C$ | 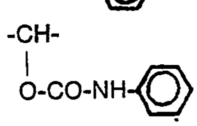 | H | | 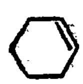 | Fp: 159°C |
| 23 | $(CH_3)_3C$ | 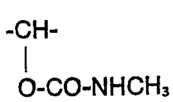 | H | $CH_3$ | $CH_3$ | Fp: 178°C |

| Beispiel Nr. | $R^1$ | X | R | $R^2$ | $R^3$ | Physikalische Konstante |
|---|---|---|---|---|---|---|
| 24 | $(CH_3)_3C$ | -CH(OH)- | H | $CH_3$ | $CH_3$ | Kp: 150°C /0,1 mmHg |
| 25 | $(CH_3)_3C$ | -CO- | H | $CH_3$ | 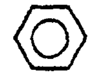 | oil |
| 26 | $ClCH_2$-$C(CH_3)_2$- | -CO- | H | $CH_3$ | $C_2H_5$ | oil |
| 27 | $ClCH_2$-$C(CH_3)_2$- | -CH(OH)- | H | $CH_3$ | $C_2H_5$ | oil |
| 28 | $ClCH_2$-$C(CH_3)_2$- | -CH(OH)- | H | $CH_3$ | 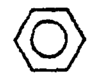 | oil |
| 29 | $ClCH_2$-$C(CH_3)_2$- | -CO- | H | $CH_3$ | 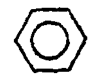 | oil |
| 30 | $(CH_3)_3C$ | -CH(OH)- | H | $CH_3$ | 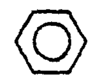 | $n_D^{20}$: 1.5427 |

**Patentansprüche**

1. 1-Allyl-triazol-Derivate der allgemeinen Formel

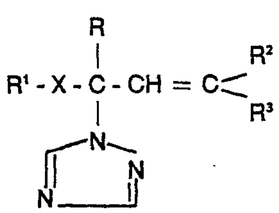

(I)

in welcher

R für Wasserstoff, Alkyl mit 1 bis 4 Kohlenstoffatomen oder für gegebenenfalls durch Halogen substituiertes Benzyl steht;

$R^1$ für Alkyl mit 1 bis 4 Kohlenstoffatomen steht, welches gegebenenfalls substituiert sein kann durch Halogen, Alkylcarbonyloxy- und Alkylsulfonyloxy-Gruppen mit jeweils 1 bis 4 Kohlenstoffatomen im Alkylteil, sowie durch die Phenylsulfonyloxy-Gruppe, wobei letztere selbst durch Alkyl mit 1 bis 4 Kohlenstoffatomen substituiert sein kann; ausserdem für Cycloalkyl mit 5 bis 7 Kohlenstoffatomen und für Phenyl steht, welches substituiert sein kann durch Halogen, Alkyl mit 1 bis 4 Kohlenstoffatomen, Phenyl, Phenoxy, Halogenphenyl und Halogenphenoxy;

$R^2$ für Alkyl mit 1 bis 4 Kohlenstoffatomen steht;

$R^3$ für Alkyl mit 1 bis 4, Cycloalkyl und Cycloalkenyl mit jeweils 5 bis 7 Kohlenstoffatomen steht, wobei letzteres durch Alkyl mit 1 bis 4 Kohlenstoffatomen substituiert sein kann, ferner für Alkenyl mit 2 bis 4 Kohlenstoffatomen und für Phenyl steht, wobei letzteres durch Halogen und Alkyl mit 1 bis 4 Kohlenstoffatomen substituiert sein kann;

$R^2$ und $R^3$ können weiterhin gemeinsam mit dem Kohlenstoffatom, an das sie gebunden sind, für gegebenenfalls durch Alkyl mit 1 bis 4 Kohlenstoffatomen substituiertes Cycloalkenyl mit 5 bis 7 Kohlenstoffatomen und für Cycloalkyl mit 3 bis 7 Kohlenstoffatomen stehen;

X für die Gruppe -$C(OR^4)R^5$- und für die Ketogruppe steht, wobei

$R^4$ für Wasserstoff, für Alkyl mit 1 bis 4 Kohlenstoffatomen sowie für Benzyl und für Naphthylmethyl steht, wobei die beiden letztgenannten Gruppen durch Halogen, durch Alkyl mit 1 bis 4 Kohlenstoffatomen, durch Phenyl, Phenoxy, Halogenphenyl und Halogenphenoxy substituiert sein können; weiterhin für den Acylrest -CO-$R^{10}$ und den Carbamoylrest -CO-$NR^{11}R^{12}$ steht;

$R^5$ für Wasserstoff, für Alkyl mit 1 bis 4 Kohlenstoffatomen, sowie für gegebenenfalls durch Halogen substituiertes Benzyl steht;

$R^{10}$ für Alkyl mit 1 bis 4 Kohlenstoffatomen, Halogenalkyl mit 1 bis 2 Kohlenstoff- und 1 bis 5 Halogenatomen und für Phenyl und Benzyl steht, wobei die beiden letztgenannten Gruppen durch Halogen und Alkyl mit 1 bis 4 Kohlenstoffatomen substituiert sein können;

$R^{11}$ für Wasserstoff und Alkyl mit 1 bis 4 Kohlenstoffatomen steht; und

$R^{12}$ für Alkyl mit 1 bis 8 Kohlenstoffatomen, Halogenalkyl mit bis zu 4 Kohlenstoff- und bis zu 5 Halogenatomen und für gegebenenfalls halogen-substituiertes Phenyl steht, und schliesslich für Halogenalkylmercapto-Gruppen mit bis zu 2 Kohlenstoff- und bis zu 5 Halogenatomen steht.

2. Verfahren zur Herstellung von Allyl-triazol-Derivaten der allgemeinen Formel

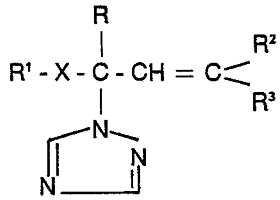

(I)

in welcher

R für Wasserstoff, Alkyl mit 1 bis 4 Kohlenstoffatomen oder für gegebenenfalls durch Halogen substituiertes Benzyl steht;

$R^1$ für Alkyl mit 1 bis 4 Kohlenstoffatomen steht, welches gegebenenfalls substituiert sein kann durch Halogen, Alkylcarbonyloxy- und Alkylsulfonyloxy-Gruppen mit jeweils 1 bis 4 Kohlenstoffatomen im Alkylteil, sowie durch die Phenylsulfonyloxy-Gruppe, wobei letztere selbst durch Alkyl mit 1 bis 4 Kohlenstoffatomen substituiert sein kann; ausserdem für Cycloalkyl mit 5 bis 7 Kohlenstoffatomen und für Phenyl steht, welches substituiert sein kann durch Halogen, Alkyl mit 1 bis 4 Kohlenstoffatomen, Phenyl, Phenoxy, Halogenphenyl und Halogenphenoxy;

$R^2$ für Alkyl mit 1 bis 4 Kohlenstoffatomen steht;

$R^3$ für Alkyl mit 1 bis 4, Cycloalkyl und Cycloalkenyl mit jeweils 5 bis 7 Kohlenstoffatomen steht, wobei letzteres durch Alkyl mit 1 bis 4 Kohlenstoffatomen substituiert sein kann, ferner für Alkenyl mit 2 bis 4 Kohlenstoffatomen und für Phenyl steht, wobei letzteres durch Halogen und Alkyl mit 1 bis 4 Kohlenstoffatomen substituiert sein kann;

$R^2$ und $R^3$ können weiterhin gemeinsam mit dem Kohlenstoffatom, an das sie gebunden sind, für gegebenenfalls durch Alkyl mit 1 bis 4 Kohlenstoffatomen substituiertes Cycloalkenyl mit 5 bis 7 Kohlenstoffatomen und für Cycloalkyl mit 3 bis 7 Kohlenstoffatomen stehen;

X für die Gruppe $-C(OR^4)R^5-$ und für die Ketogruppe steht, wobei

$R^4$ für Wasserstoff, für Alkyl mit 1 bis 4 Kohlenstoffatomen sowie für Benzyl und für Naphthylmethyl steht, wobei die beiden letztgenannten Gruppen durch Halogen, durch Alkyl mit 1 bis 4 Kohlenstoffatomen, durch Phenyl, Phenoxy, Halogenphenyl und Halogenphenoxy substituiert sein können; weiterhin für den Acylrest $-CO-R^{10}$ und den Carbamoylrest $-CO-NR^{11}R^{12}$ steht;

$R^5$ für Wasserstoff, für Alkyl mit 1 bis 4 Kohlenstoffatomen, sowie für gegebenenfalls durch Halogen substituiertes Benzyl steht;

$R^{10}$ für Alkyl mit 1 bis 4 Kohlenstoffatomen, Halogenalkyl mit 1 bis 2 Kohlenstoff- und 1 bis 5 Halogenatomen und für Phenyl und Benzyl steht, wobei die beiden letztgenannten Gruppen durch Halogen und Alkyl mit 1 bis 4 Kohlenstoffatomen substituiert sein können;

$R^{11}$ für Wasserstoff und Alkyl mit 1 bis 4 Kohlenstoffatomen steht; und

$R^{12}$ für Alkyl mit 1 bis 8 Kohlenstoffatomen, Halogenalkyl mit bis zu 4 Kohlenstoff- und bis zu 5 Halogenatomen und für gegebenenfalls halogen-substituiertes Phenyl steht, und schliesslich für Halogenalkylmercapto-Gruppen mit bis zu 2 Kohlenstoff- und bis zu 5 Halogenatomen steht,

dadurch gekennzeichnet, dass man

a) Triazol-Ketone der Formel

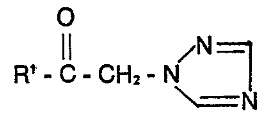

(II)

in welcher

$R^1$ die oben angegebene Bedeutung hat, mit Aldehyden der Formel

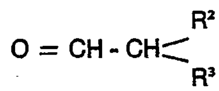

(III)

in welcher

$R^2$ und $R^3$ die oben angegebene Bedeutung haben,

in Gegenwart eines Lösungsmittels und in Gegenwart eines Katalysators umsetzt und von den aufgrund der Wasserabspaltung sich bildenden Isomeren nach üblichen Methoden das gewünschte isomere Produkt der Formel

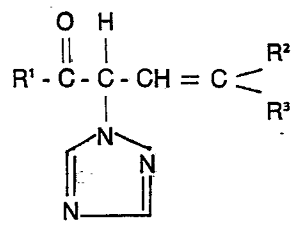

(Ia)

in welcher

$R^1$, $R^2$ und $R^3$ die oben angegebene Bedeutung, haben, isoliert, oder

b) die nach Verfahren (a) erhältlichen Verbindungen der Formel (Ia) in an sich bekannter Art und Weise

α) mit komplexen Hydriden in Gegenwart eines Lösungsmittels reduziert, oder

β) mit einer Grignard-Verbindung der Formel

$$R^6 - Mg - Hal \quad (IV)$$

in welcher

$R^6$ die oben unter $R^5$ genannten Bedeutungen mit Ausnahme von Wasserstoff besitzt und

Hal für Halogen steht,

in Gegenwart eines Lösungsmittels umsetzt, oder die nach Verfahren (b) erhältlichen Verbindungen der Formel

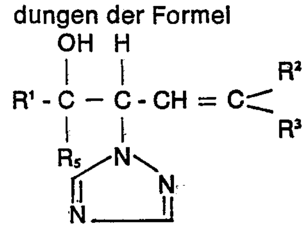

(Ib)

in welcher
$R^1$, $R^2$, $R^3$ und $R^5$ die weiter oben genannten Bedeutungen besitzen,
c) mit Halogeniden der Formel

$$R^7 - Hal \qquad (V)$$

in welcher
$R^7$ die oben unter $R^4$ genannten Bedeutungen mit Ausnahme von Wasserstoff besitzt, und
Hal für Halogen steht,
in Gegenwart eines Lösungsmittels und gegebenenfalls in Gegenwart einer starken Base bzw. gegebenenfalls in Gegenwart eines Säurebindemittels umsetzt, oder
d) mit Säureanhydriden der Formel

$$R^8 - O - R^8 \qquad (VI)$$

in welcher
$R^8$ für den Azylrest -CO-$R^{10}$ steht, wobei
$R^{10}$ die oben angegeben Bedeutungen besitzt, in Gegenwart eines Lösungsmittels und gegebenenfalls in Gegenwart eines Katalysators umsetzt, oder
e) mit Isocyanaten der Formel

$$O = C = N - R^9 \qquad (VII)$$

in welcher
$R^9$ für die oben unter $R^{12}$ genannten Bedeutungen mit Ausnahme von Halogenalkylmercapto steht,
in Gegenwart eines Lösungsmittels und gegebenenfalls in Gegenwart eines Katalysators umsetzt, oder
f) die nach Verfahren (a) erhältlichen Verbindungen der Formel (Ia) mit Halogeniden der Formel

$$Y - Hal \qquad (VIII)$$

in welcher
Y die oben unter R genannten Bedeutungen mit Ausnahme von Wasserstoff besitzt,
in Gegenwart einer starken Base und in Gegenwart eines Lösungsmittels umsetzt und
gegebenenfalls die erhaltenen Verbindungen der Formel

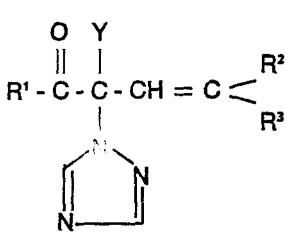

(Iaa)

in welcher
$R^1$, $R^2$, $R^3$ und Y die oben angegebene Bedeutung haben,
entsprechend den Verfahren (b), (c), (d) und (e) weiter umsetzt,
und gegebenenfalls anschliessend eine Säure oder ein Metallsalz addiert.

3. Pflanzenschutzmittel, gekennzeichnet durch einen Gehalt an mindestens einem 1-Allyl-triazol-Derivat der Formel I in Ansprüchen 1 und 2.

4. Verwendung von 1-Allyl-triazol-Derivaten der Formel I in Ansprüchen 1 und 2 zur Bekämpfung von Pilzen.

5. Verwendung von 1-Allyl-triazol-Derivaten der Formel I in Ansprüchen 1 und 2 zur Regulierung des Pflanzenwachstums.

6. Verfahren zur Herstellung von Pflanzenschutzmitteln, dadurch gekennzeichnet, dass man 1-Allyl-triazol-Derivate der Formel I in Ansprüchen 1 und 2 mit Streckmitteln und/oder oberflächenaktiven Mitteln vermischt.

**Claims**

1. 1-Allyl-triazole derivatives of the general formula

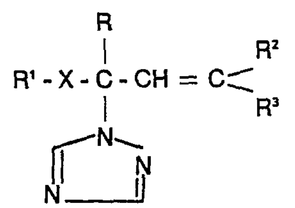

(I)

in which
R represents hydrogen, alkyl with 1 to 4 carbon atoms or benzyl which is optionally substituted by halogen;
$R^1$ represents alkyl with 1 to 4 carbon atoms, which can optionally be substituted by halogen, alkylcarbonyloxy and alkylsulphonyloxy groups with in each case 1 to 4 carbon atoms in the alkyl part, and by the phenylsulphonyloxy group, which can itself be substituted by alkyl with 1 to 4 carbon atoms; and also represents cycloalkyl with 5 to 7 carbon atoms and phenyl which can be substituted by halogen, alkyl with 1 to 4 carbon atoms, phenyl, phenoxy, halogenophenyl and halogenophenoxy;
$R^2$ represents alkyl with 1 to 4 carbon atoms;
$R^3$ represents alkyl with 1 to 4 carbon atoms, cycloalkyl and cycloalkenyl with in each case 5 to 7 carbon atoms, it being possible for the latter to be substituted by alkyl with 1 to 4 carbon atoms, and furthermore represents alkenyl with 2 to 4 carbon atoms and phenyl, it being possible for the latter to be substituted by halogen and alkyl with 1 to 4 carbon atoms;
$R^2$ and $R^3$ can furthermore, together with the carbon atom to which they are bonded, represent cycloalkenyl with 5 to 7 carbon atoms, which is optionally substituted by alkyl with 1 to 4 carbon atoms, and cycloalkyl with 3 to 7 carbon atoms;
X represents the group -C($OR^4$)$R^5$- and the keto group, wherein
$R^4$ represents hydrogen, alkyl with 1 to 4 carbon atoms and benzyl and naphthylmethyl, it being possible for the two last-mentioned

groups to be substituted by halogen, alkyl with 1 to 4 carbon atoms, by phenyl, phenoxy, halogenophenyl and halogenophenoxy; and furthermore represents the acyl radical $-CO-R^{10}$ and the carbamoyl radical $-CO-NR^{11}R^{12}$;

$R^5$ represents hydrogen, alkyl with 1 to 4 carbon atoms, and benzyl which is optionally substituted by halogen;

$R^{10}$ represents alkyl with 1 to 4 carbon atoms, halogenoalkyl with 1 to 2 carbon atoms and 1 to 5 halogen atoms and phenyl and benzyl, it being possible for the two last-mentioned groups to be substituted by halogen and alkyl with 1 to 4 carbon atoms;

$R^{11}$ represents hydrogen and alkyl with 1 to 4 carbon atoms; and

$R^{12}$ represents alkyl with 1 to 8 carbon atoms, halogenoalkyl with up to 4 carbon atoms and up to 5 halogen atoms and optionally halogen-substituted phenyl, and finally represents halogenoalkylmercapto groups with up to 2 carbon atoms and up to 5 halogen atoms.

2. Process for the preparation of allyl-triazole derivatives of the general formula

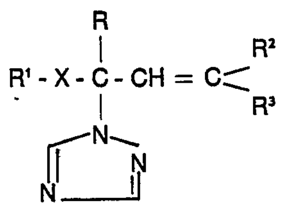

(I)

in which

R represents hydrogen, alkyl with 1 to 4 carbon atoms or benzyl which is optionally substituted by halogen;

$R^1$ represents alkyl with 1 to 4 carbon atoms, which can optionally be substituted by halogen, alkylcarbonyloxy and alkylsulphonyloxy groups with in each case 1 to 4 carbon atoms in the alkyl part, and by the phenylsulphonyloxy group, which can itself be substituted by alkyl with 1 to 4 carbon atoms; and also represents cycloalkyl with 5 to 7 carbon atoms and phenyl which can be substituted by halogen, alkyl with 1 to 4 carbon atoms, phenyl, phenoxy, halogenophenyl and halogenophenoxy;

$R^2$ represents alkyl with 1 to 4 carbon atoms;

$R^3$ represents alkyl with 1 to 4 carbon atoms, cycloalkyl and cycloalkenyl with in each case 5 to 7 carbon atoms, it being possible for the latter to be substituted by alkyl with 1 to 4 carbon atoms, and furthermore represents alkenyl with 2 to 4 carbon atoms and phenyl, it being possible for the latter to be substituted by halogen and alkyl with 1 to 4 carbon atoms;

$R^2$ and $R^3$ can furthermore, together with the carbon atom to which they are bonded, represent cycloalkenyl with 5 to 7 carbon atoms, which is optionally substituted by alkyl with 1 to 4 carbon atoms, and cycloalkyl with 3 to 7 carbon atoms;

X represents the group $-C(OR^4)R^5-$ and the keto group, wherein

$R^4$ represents hydrogen, alkyl with 1 to 4 carbon atoms and benzyl and naphthylmethyl, it being possible for the two last-mentioned groups to be substituted by halogen, alkyl with 1 to 4 carbon atoms, by phenyl, phenoxy, halogenophenyl and halogenophenoxy; and furthermore represents the acyl radical $-CO-R^{10}$ and the carbamoyl radical $-CO-NR^{11}R^{12}$;

$R^5$ represents hydrogen, alkyl with 1 to 4 carbon atoms, and benzyl which is optionally substituted by halogen;

$R^{10}$ represents alkyl with 1 to 4 carbon atoms, halogenoalkyl with 1 to 2 carbon atoms and 1 to 5 halogen atoms and phenyl and benzyl, it being possible for the two last-mentioned groups to be substituted by halogen and alkyl with 1 to 4 carbon atoms;

$R^{11}$ represents hydrogen and alkyl with 1 to 4 carbon atoms; and

$R^{12}$ represents alkyl with 1 to 8 carbon atoms, halogenoalkyl with up to 4 carbon atoms and up to 5 halogen atoms and optionally halogen-substituted phenyl, and finally represents halogenoalkylmercapto groups with up to 2 carbon atoms and up to 5 halogen atoms,

characterised in that

a) triazole-ketones of the formula

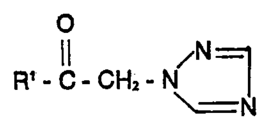

(II)

in which

$R^1$ has the meaning indicated above,

are reacted with aldehydes of the formula

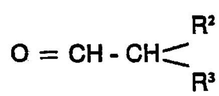

(III)

in which

$R^2$ and $R^3$ have the meaning indicated above, in the presence of a solvent and in the presence of a catalyst, and from the isomers which form as a result of splitting off of water, the desired isomeric product of the formula

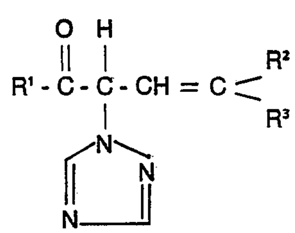

(Ia)

in which

$R^1$, $R^2$ and $R^3$ have the meaning indicated above, is isolated by cutomary methods, or

b) the compounds of the formula (Ia) obtainable by process (a) are, in a manner which is in itself known,

α) reduced with complex hydrides in the presence of a solvent or

β) reacted with a Grignard compound of the formula

$$R^6 - Mg - Hal \qquad (IV)$$

in which
$R^6$ has the meanings given above under $R^5$ with the exception of hydrogen and
Hal represents halogen,
in the presence of a solvent, or the compounds obtainable by process (b), of the formula

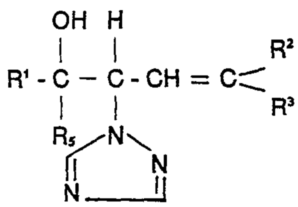

(Ib)

in which
$R^1$, $R^2$, $R^3$ and $R^5$ have the meanings indicated further above
c) reacted with halides of the formula

$$R^7 - Hal \qquad (V)$$

in which
$R^7$ has the meanings indicated under $R^4$ with the exception of hydrogen and
Hal represents halogen,
in the presence of a solvent and if appropriate in the presence of a strong base or if appropriate in the presence of an acid-binding agent, or
d) are reacted with acid anhydrides of the formula

$$R^8 - O - R^8 \qquad (VI)$$

in which
$R^8$ represents the acyl radical $-CO-R^{10}$, wherein
$R^{10}$ has the meanings given above,
in the presence of a solvent and if appropriate in the presence of a catalyst, or
e) are reacted with isocyanates of the formula

$$O = C = N - R^9 \qquad (VII)$$

in which
$R^9$ has the meanings indicated above under $R^{12}$ with the exception of halogenoalkylmercapto,
in the presence of a solvent and if appropriate in the presence of a catalyst, or
f) the compounds of formula (Ia) obtainable by process (a) are reacted with halides of the formula

$$Y - Hal \qquad (VIII)$$

in which
Y has the meanings indicated above under R with the exception of hydrogen
in the presence of a strong base and in the presence of a solvent and the resulting compounds of the formula

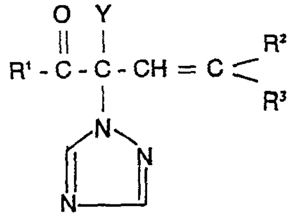

(Iaa)

in which
$R^1$, $R^2$, $R^3$ and Y have the meaning indicated above, are optionally reacted further according to processes (b), (c), (d) and (e), and an acid or a metal salt is then optionally added on.

3. Plant protection agents, characterised in that they contain at least one 1-allyl-triazole derivative of the formula I in claims 1 and 2.

4. Use of 1-allyl-triazole derivatives of the formula I in claims 1 and 2 for combating fungi.

5. Use of 1-allyl-triazole derivatives of the formula I in claims 1 and 2 for regulating plant growth.

6. Process for the preparation of plant protection agents, characterised in that 1-allyl-triazole derivatives of the formula I in claims 1 and 2 are mixed with extenders and/or surface-active agents.

**Revendications**

1. Dérivés de 1-allyl-triazole de formule générale:

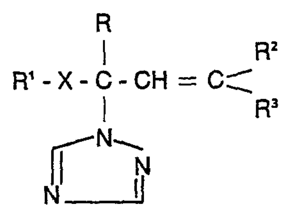

(I)

dans laquelle
R représente un atome d'hydrogène, un groupe alkyle ayant 1 à 4 atomes de carbone ou un groupe benzyle éventuellement substitué par de l'halogène;
$R^1$ représente un groupe alkyle ayant 1 à 4 atomes de carbone (qui peut éventuellement être substitué par de l'halogène, par des groupes alkylcarbonyloxy et alkylsulfonyloxy ayant chacun 1 à 4 atomes de carbone dans la partie alkyle, ainsi que par le groupe phénylsulfonyloxy, ce dernier pouvant lui-même être substitué par un groupe alkyle ayant 1 à 4 atomes de carbone), ainsi qu'un groupe cycloalkyle ayant 5 à 7 atomes de carbone et un groupe phényle (lequel peut être substitué par de l'halogène, par un groupe alkyle ayant 1 à 4 atomes de carbone, phényle, phénoxy, halogénophényle et halogénophénoxy);
$R^2$ représente un groupe alkyle ayant 1 à 4 atomes de carbone;
$R^3$ représente un groupe alkyle ayant 1 à 4 atomes de carbone, cycloalkyle et cycloalcényle ayant chacun 5 à 7 atomes de carbone (ce

dernier pouvant être substitué par un groupe alkyle ayant 1 à 4 atomes de carbone), ainsi qu'un groupe alcényle ayant 2 à 4 atomes de carbone et un groupe phényle (ce dernier pouvant être substitué par de l'halogène et par un groupe alkyle ayant 1 à 4 atomes de carbone);

$R^2$ et $R^3$ peuvent en outre représenter, lorsqu'ils sont pris avec l'atome de carbone auquel ils sont liés, un groupe cycloalcényle ayant 5 à 7 atomes de carbone (éventuellement substitué par un groupe alkyle ayant 1 à 4 atomes de carbone) et un groupe cycloalkyle ayant 3 à 7 atomes de carbone;

X représente le groupe $-C(OR^4)R^5-$ et le groupe cétone, où

$R^4$ représente un atome d'hydrogène, un groupe alkyle ayant 1 à 4 atomes de carbone ainsi qu'un groupe benzyle et un groupe naphthylméthyle (ces deux derniers groupes pouvant être substitués par de l'halogène, par un groupe alkyle ayant 1 à 4 atomes de carbone, par un groupe phényle, phénoxy, halogénophényle et halogénophénoxy), ainsi qu'un radical acyle $-CO-R^{10}$ et le radical carbamoyle $-CO-NR^{11}R^{12}$;

$R^5$ représente un atome d'hydrogène, un groupe alkyle ayant 1 à 4 atomes de carbone, ainsi qu'un groupe benzyle (éventuellement substitué par de l'halogène);

$R^{10}$ représente un groupe alkyle ayant 1 à 4 atomes de carbone, halogénoalkyle ayant 1 à 2 atomes de carbone et 1 à 5 atomes d'halogène et un groupe phényle et benzyle (ces deux derniers groupes pouvant être substitué par de l'halogène et par un groupe alkyle ayant 1 à 4 atomes de carbone);

$R^{11}$ représente un atome d'hydrogène et un groupe alkyle ayant 1 à 4 atomes de carbone; et

$R^{12}$ représente un groupe alkyle ayant 1 à 8 atomes de carbone, halogénoalkyle ayant jusqu'à 4 atomes de carbone et jusqu'à 5 atomes d'halogène et un groupe phényle éventuellement substitué par de l'halogène, et enfin des groupes halogénoalkylmercapto ayant jusqu'à 2 atomes de carbone et jusqu'à 5 atomes d'halogène.

2. Procédé de production de dérivés d'allyltriazole de formule générale

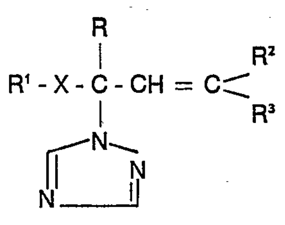

(I)

[dans laquelle

R représente un atome d'hydrogène, un groupe alkyle ayant 1 à 4 atomes de carbone ou un groupe benzyle éventuellement substitué par de l'halogène;

$R^1$ représente un groupe alkyle ayant 1 à 4 atomes de carbone (qui peut éventuellement être substitué par de l'halogène, par des groupes alkylcarbonyloxy et alkylsulfonyloxy ayant chacun 1 à 4 atomes de carbone dans la partie alkyle, ainsi que par le groupe phénylsulfonyloxy, ce dernier pouvant lui-même être substitué par un groupe alkyle ayant 1 à 4 atomes de carbone), ainsi qu'un groupe cycloalkyle ayant 5 à 7 atomes de carbone et un groupe phényle (lequel peut être substitué par de l'halogène, par un groupe alkyle ayant 1 à 4 atomes de carbone, phényle, phénoxy, halogénophényle et halogénophénoxy);

$R^2$ représente un groupe alkyle ayant 1 à 4 atomes de carbone;

$R^3$ représente un groupe alkyle ayant 1 à 4 atomes de carbone, cycloalkyle et cycloalcényle ayant chacun 5 à 7 atomes de carbone (ce dernier pouvant être substitué par un groupe alkyle ayant 1 à 4 atomes de carbone), ainsi qu'un groupe alcényle ayant 2 à 4 atomes de carbone et un groupe phényle (ce dernier pouvant être substitué par de l'halogène et par un groupe alkyle ayant 1 à 4 atomes de carbone);

$R^2$ et $R^3$ peuvent en outre représenter, lorsqu'ils sont pris avec l'atome de carbone auquel ils sont liés, un groupe cycloalcényle ayant 5 à 7 atomes de carbone (éventuellement substitué par un groupe alkyle ayant 1 à 4 atomes de carbone) et un groupe cycloalkyle ayant 3 à 7 atomes de carbone;

X représente le groupe $-C(OR^4)R^5-$ et le groupe cétone, où

$R^4$ représente un atome d'hydrogène, un groupe alkyle ayant 1 à 4 atomes de carbone ainsi qu'un groupe benzyle et un groupe naphthylméthyle (ces deux derniers groupes pouvant être substitués par de l'halogène, par un groupe alkyle ayant 1 à 4 atomes de carbone, par un groupe phényle, phénoxy, halogénophényle et halogénophénoxy), ainsi qu'un radical acyle $-CO-R^{10}$ et le radical carbamoyle $-CO-NR^{11}R^{12}$;

$R^5$ représente un atome d'hydrogène, un groupe alkyle ayant 1 à 4 atomes de carbone, ainsi qu'un groupe benzyle (éventuellement substitué par de l'halogène);

$R^{10}$ représente un groupe alkyle ayant 1 à 4 atomes de carbone, halogénoalkyle ayant 1 à 2 atomes de carbone et 1 à 5 atomes d'halogène et un groupe phényle et benzyle (ces deux derniers groupes pouvant être substitué par de l'halogène et par un groupe alkyle ayant 1 à 4 atomes de carbone);

$R^{11}$ représente un atome d'hydrogène et un groupe alkyle ayant 1 à 4 atomes de carbone; et

$R^{12}$ représente un groupe alkyle ayant 1 à 8 atomes de carbone, halogénoalkyle ayant jusqu'à 4 atomes de carbone et jusqu'à 5 atomes d'halogène et un groupe phényle éventuellement substitué par de l'halogène, et enfin des groupes halogénoalkylmercapto ayant jusqu'à 2 atomes de carbone et jusqu'à 5 atomes d'halogène],

caractérisé en ce que:

a) on fait réagir des triazolo-cétones de formule:

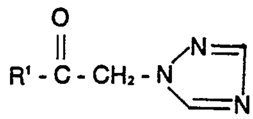

(II)

(dans laquelle
$R^1$ a le sens indiqué ci-dessus)
avec des aldéhydes de formule:

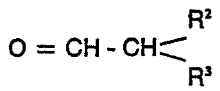

(III)

(dans laquelle
$R^2$ et $R^3$ ont le sens indiqué ci-dessus)
en présence d'un solvant et en présence d'un catalyseur et l'on isole par des méthodes usuelles, à partir des isomères qui se forment par séparation de l'eau, le produit isomère voulu de formule:

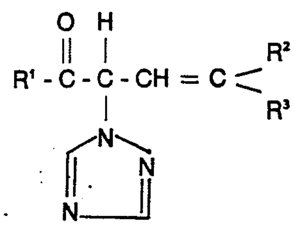

(Ia)

(dans laquelle
$R^1$, $R^3$ et $R^2$ ont le sens indiqué ci-dessus), ou

b) on soumet, de façon connue en soi, les composés de formule (Ia) que l'on peut obtenir selon le procédé a):
   α) à une réduction à l'aide d'hydrures complexes en présence d'un solvant, ou
   β) à une réaction avec un composé de Grignard de formule

$$R^6 - Mg - Hal \qquad (IV)$$

(dans laquelle
$R^6$ a le sens indiqué ci-dessus pour $R^5$, à l'exclusion de l'hydrogène; et
Hal représente un halogène)
en présence d'un solvant, ou on soumet les composés de formule:

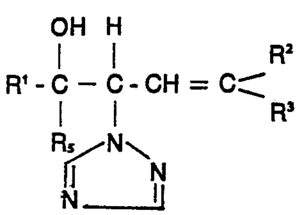

(Ib)

(dans laquelle
$R^1$, $R^2$, $R^3$ et $R^5$ ont les sens indiqué ci-dessus), que l'on peut obtenir selon le procédé b),

c) à une réaction avec des halogénures de formule

$$R^7 - Hal \qquad (V)$$

(dans laquelle
$R^7$ a le sens indiqué ci-dessus pour $R^4$, à l'exclusion de l'hydrogène; et
Hal représente un halogène),
en présence d'un solvant et éventuellement en présence d'une base forte ou éventuellement en présence d'un agent de fixation des acides, ou

d) à une réaction avec des anhydrides d'acide de formule

$$R^8 - O - R^8 \qquad (VI)$$

(dans laquelle
$R^8$ représente le reste acyle $-CO-R^{10}$, où
$R^{10}$ a les sens précités),
en présence d'un solvant et éventuellement en présence d'un catalyseur, ou

e) à une réaction avec des isocyanates de formule

$$O = C = N - R^9 \qquad (VII)$$

(dans laquelle
$R^9$ a les sens indiqués ci-dessus pour $R^{12}$, à l'exclusion d'un groupe halogénoalkylmercapto), en présence d'un solvant et éventuellement en présence d'un catalyseur, ou

f) on fait réagir les composés de formule a), que l'on peut obtenir selon le procédé a), avec des halogénures de formule

$$Y - Hal \qquad (VIII)$$

(dans laquelle
Y a les sens indiqués ci-dessus pour R, à l'exclusion de l'hydrogène),
en présence d'une base forte et en présence d'un solvant, et éventuellement on fait encore réagir selon les procédé b), c), d) et e) les composés obtenus, de formule

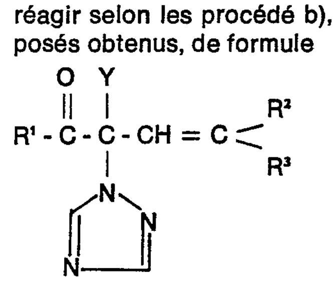

(Iaa)

(dans laquelle
$R^1$, $R^2$, $R^3$ et Y ont le sens indiqué ci-dessus),
et éventuellement on fixe ensuite par addition un acide ou un sel de métal.

3. Produit phytopharmaceutique, caractérisé en ce qu'il contient au moins un dérivé de 1-allyl-triazole de formule (I) selon les revendications 1 et 2.

4. Application des dérivés de 1-allyl-triazole de formule (I), selon les revendications 1 et 2, à la lutte contre les champignons.

5. Application des dérivés de 1-allyl-triazole de formule (I), selon les revendications 1 et 2, à la régulation de la croissance des plantes.

6. Procédé de production d'un produit phytopharmaceutique, caractérisé en ce qu'on mélange des dérivés de 1-allyl-triazole de formule (I), selon les revendications 1 et 2, avec des agents d'allongement et/ou avec des agents tensioactifs.